(19) Europäisches Patentamt
European Patent Office
Office européen des brevets

(11) **EP 3 181 203 A1**

(12) **EUROPEAN PATENT APPLICATION**
published in accordance with Art. 153(4) EPC

(43) Date of publication:
**21.06.2017 Bulletin 2017/25**

(51) Int Cl.:
**A63B 69/36** (2006.01)

(21) Application number: **15831526.7**

(22) Date of filing: **06.08.2015**

(86) International application number:
**PCT/JP2015/003953**

(87) International publication number:
**WO 2016/024392 (18.02.2016 Gazette 2016/07)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **11.08.2014 JP 2014163897**

(71) Applicant: **Seiko Epson Corporation
Tokyo 160-8801 (JP)**

(72) Inventors:
• **SAYO, Takefumi**
  **Suwa-shi**
  **Nagano 392-8502 (JP)**
• **ITO, Tsuyoshi**
  **Suwa-shi**
  **Nagano 392-8502 (JP)**

(74) Representative: **Hoffmann Eitle
Patent- und Rechtsanwälte PartmbB
Arabellastraße 30
81925 München (DE)**

(54) **MOTION ANALYSIS METHOD, MOTION ANALYSIS DEVICE, MOTION ANALYSIS SYSTEM, AND PROGRAM**

(57)     A motion analysis method, a motion analysis apparatus, a motion analysis system, and a program, capable of reducing time and effort to perform work of editing captured images are provided.

A motion analysis method includes an action detection step (S100) of detecting an action in motion of a user 2 by using an output signal from a sensor unit 10, an image data acquisition step (S110) of acquiring image data of the motion of the user 2, captured by an imaging apparatus 30, and an analysis information generation step (S120) of correlating the acquired image data with the detected action.

FIG. 7

**Description**

Technical Field

The present invention relates to a motion analysis method, a motion analysis apparatus, a motion analysis system, and a program.

Background Art

PTL 1 discloses an apparatus in which a three-axis acceleration sensor and a three-axis gyro sensor are attached to a golf club, and a golf swing is analyzed.

Citation List

Patent Literature

PTL 1: JP-A-2008-73210

Summary of Invention

Technical Problem

However, in the swing analysis apparatus employing the inertial sensors as disclosed in PTL 1, there is a case where a user is not satisfied when viewing a swing analysis result, for example, when the user feels that an actual swing of the user does not match a displayed image of a swing trajectory. Therefore, a service in which a swing analysis result is combined with moving images obtained by imaging a user' s swing has started to be examined. For example, a technique has been examined in which a user captures moving images of the user's swing with a smart phone or a tablet PC, the captured moving images are displayed to overlap a trajectory which is calculated on the basis of an output of a sensor, and thus the user can easily view a swing analysis result. However, in a case where a user desires to perform work of editing captured images, for example, it is not easy to accurately specify an image of a characteristic moment such as a top or impact in a swing, the work of editing captured images may require considerable time and effort, and thus there is a problem of inconvenience.

The present invention has been made in consideration of the above-described problems, and, according to some aspects of the present invention, it is possible to provide a motion analysis method, a motion analysis apparatus, a motion analysis system, and a program, capable of reducing time and effort to perform work of editing captured images.

Solution to Problem

The present invention has been made in order to solve at least a part of the above-described problems, and can be realized in the following aspects or application examples.

[Application Example 1]

A motion analysis method according to this application example is a motion analysis method including an action detection step of detecting an action in motion of a user by using an output signal from an inertial sensor; an image data acquisition step of acquiring image data of the motion of the user, captured by imaging means; and an analysis information generation step of correlating the image data with the detected action.

The inertial sensor may be a sensor which can measure an inertia amount such as acceleration or angular velocity, and may be, for example, an inertial measurement unit (IMU) which can measure acceleration or angular velocity. The inertial sensor is attached to, for example, an external apparatus or a part of a user, may be attachable to and detachable from an exercise appliance or a user, and may be fixed to an exercise appliance so as not to be detachable therefrom as a result of being built into the exercise appliance.

According to the motion analysis method of the application example, it is possible to specify captured image data in correlation with an action in the motion of the user, and thus to reduce time and effort to perform work of editing captured images.

[Application Example 2]

In the motion analysis method according to the application example, synchronization between the output signal from the inertial sensor and the image data captured by the imaging means may occur.

[Application Example 3]

In the motion analysis method according to the application example, the synchronization may occur on the basis of a timing of a first state in the motion, detected on the basis of the output signal from the inertial sensor.

[Application Example 4]

In the motion analysis method according to the application example, in the analysis information generation step, a flag may be added to image data corresponding to the detected action among the image data items.

According to the motion analysis method of the application example, it is possible to specify captured image data in correlation with an action in the motion of the user on the basis of the flag.

[Application Example 5]

In the motion analysis method according to the application example, in the analysis information generation step, types of the flag may be different from each other depending on types of the detected action.

According to the motion analysis method of the application example, it is possible to easily specify captured image data in correlation with a desired action in the motion of the user on the basis of the type of flag.

[Application Example 6]

In the motion analysis method according to the application example, the motion may be swing motion using an exercise appliance.

The exercise appliance maybe a golf club, a tennis racket, a baseball bat, or a hockey stick.

According to the motion analysis method of the application example, it is possible to reduce time and effort to perform work of editing images captured in the swing motion of the user.

[Application Example 7]

In the motion analysis method according to the application example, in the action detection step, an action when the user starts the swing motion may be detected.

An action when the swing motion is started is not limited to an action at the moment of starting the swing motion, and may be an action near the moment of starting the swing motion (a predetermined range including the moment of starting the swing motion).

According to the motion analysis method of the application example, it is possible to easily specify captured image data in correlation with an action when the user starts the swing motion.

[Application Example 8]

In the motion analysis method according to the application example, in the action detection step, an action when the user switches a direction of the swing motion may be detected.

The action when a direction of the swing motion is switched is not limited to an action at the moment of switching a direction of the swing motion, and may be an action near the moment of switching a direction of the swing motion (a predetermined range including the moment of switching a direction of the swing motion).

According to the motion analysis method of the application example, it is possible to easily specify captured image data in correlation with an action when the user switches a direction of the swing.

[Application Example 9]

In the motion analysis method according to the application example, in the action detection step, an action when a speed of the swing motion is the maximum may be detected.

The action when a swing speed is the maximum is not limited to an action at the moment at which a swing speed is the maximum, and may be an action near the moment at which a swing speed is the maximum (a predetermined range including the moment at which a swing speed is the maximum).

According to the motion analysis method of the application example, it is possible to easily specify captured image data in correlation with an action when a swing speed is the maximum.

[Application Example 10]

In the motion analysis method according to the application example, in the action detection step, an action when the user hits a ball may be detected.

The action when the user hits the ball is not limited to an action at the moment at which the user hits the ball, and may be an action near the moment at which the user hits the ball (a predetermined range including the moment at which the user hits the ball).

According to the motion analysis method of the application example, it is possible to easily specify captured image data in correlation with an action when the user hits the ball.

[Application Example 11]

In the motion analysis method according to the application example, in the action detection step, an action when the user finishes the swing motion may be detected.

The action when the user finishes the swing motion is not limited to an action at the moment at which the user finishes the swing motion, and may be an action near the moment at which the user finishes the swing motion (a predetermined range including the moment at which the user finishes the swing motion).

According to the motion analysis method of the application example, it is possible to easily specify captured image data in correlation with an action when the user finishes the swing motion.

[Application Example 12]

In the motion analysis method according to the application example, the exercise appliance may be a golf club.

According to the motion analysis method of the application example, it is possible to reduce time and effort to perform work of editing images captured in the golf swing of the user.

[Application Example 13]

In the motion analysis method according to the application example, the imaging may be continuous imaging of still images.

According to the motion analysis method of the application example, it is possible to obtain a higher quality image than in a case of capturing moving images.

[Application Example 14]

A motion analysis apparatus according to this application example includes an action detection portion that detects an action in motion of a user by using an output signal from an inertial sensor; an image data acquisition portion that acquires image data of the motion of the user, captured by imaging means; and an analysis information generation portion that correlates the image data with the detected action.

According to the motion analysis apparatus of the application example, it is possible to specify captured image data in correlation with an action in the motion of the user, and thus to reduce time and effort to perform work of editing captured images.

[Application Example 15]

A motion analysis system according to this application example includes the motion analysis apparatus; and the inertial sensor.

[Application Example 16]

The motion analysis system according to the application example may further include the imaging means.

According to the motion analysis system of the application example, it is possible to specify captured image data in correlation with an action in the motion of the user, and thus to reduce time and effort to perform work of editing captured images.

[Application Example 17]

A program according to this application example causes a computer to execute an action detection step of detecting an action in motion of a user by using an output signal from an inertial sensor; an image data acquisition step of acquiring image data of the motion of the user, captured by imaging means; and an analysis information generation step of correlating the image data with the detected action.

According to the program of the application example, it is possible to specify captured image data in correlation with an action in the motion of the user, and thus to reduce time and effort to perform work of editing captured images.

Brief Description of Drawings

[Fig. 1] Fig. 1 is a diagram for explaining a summary of a motion analysis system according to a first embodiment.
[Fig. 2] Fig. 2 shows diagrams illustrating examples of positions where a sensor unit is attached.

[Fig. 3] Fig. 3 is a diagram illustrating procedures of actions performed by a user in the present embodiment.
[Fig. 4] Fig. 4 is a diagram illustrating a configuration example of the motion analysis system according to the first embodiment.
[Fig. 5] Fig. 5 is a diagram illustrating an example of imaging control performed by a processing section 21.
[Fig. 6] Fig. 6 is a diagram illustrating a correspondence relationship between image data and each action in the example illustrated in Fig. 5.
[Fig. 7] Fig. 7 is a flowchart illustrating examples of procedures of a motion analysis process in the first embodiment.
[Fig. 8] Fig. 8 is a flowchart illustrating examples of procedures of a process of detecting each action during a swing.
[Fig. 9] Fig. 9(A) is a diagram in which three-axis angular velocities during a swing are displayed in a graph, Fig. 9(B) is a diagram in which a combined value of the three-axis angular velocities is displayed in a graph, and Fig. 9(C) is a diagram in which a derivative value of the combined value of the three-axis angular velocities is displayed in a graph.
[Fig. 10] Fig. 10 is a diagram illustrating a summary of a motion analysis system according to a second embodiment.
[Fig. 11] Fig. 11 is a diagram illustrating a configuration example of the motion analysis system according to the second embodiment.
[Fig. 12] Fig. 12 is a flowchart illustrating examples of procedures of a motion analysis process in the second embodiment.

Description of Embodiments

Hereinafter, preferred embodiments of the present invention will be described with reference to the drawings. The embodiments described below are not intended to improperly limit the content of the present invention disclosed in the claims. In addition, all constituent elements described below are not essential constituent elements of the present invention.

Hereinafter, a motion analysis system (motion analysis apparatus) analyzing a golf swing will be described as an example.

1. Motion Analysis System

1-1. First embodiment

1-1-1. Summary of motion analysis system

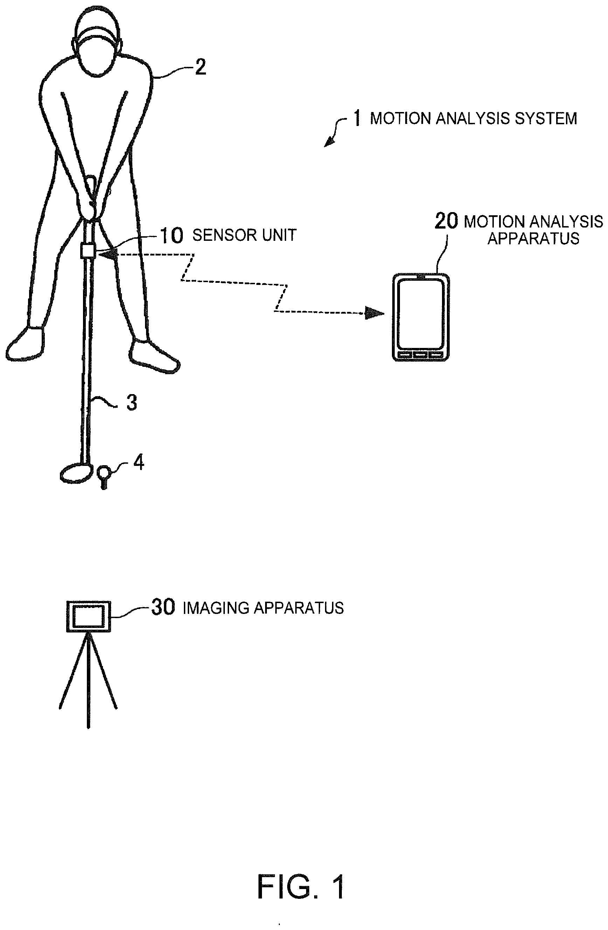

Fig. 1 is a diagram for explaining a summary of a motion analysis system according to a first embodiment. A motion analysis system 1 of the first embodiment is configured to include a sensor unit 10 (an example of an inertial sensor), a motion analysis apparatus 20, and an imaging apparatus 30 (an example of imaging means).

The sensor unit 10 can measure acceleration generated in each axial direction of three axes and angular velocity generated about each of the three axes, and is attached to a golf club 3 (an example of an exercise appliance) or a part of a user 2.

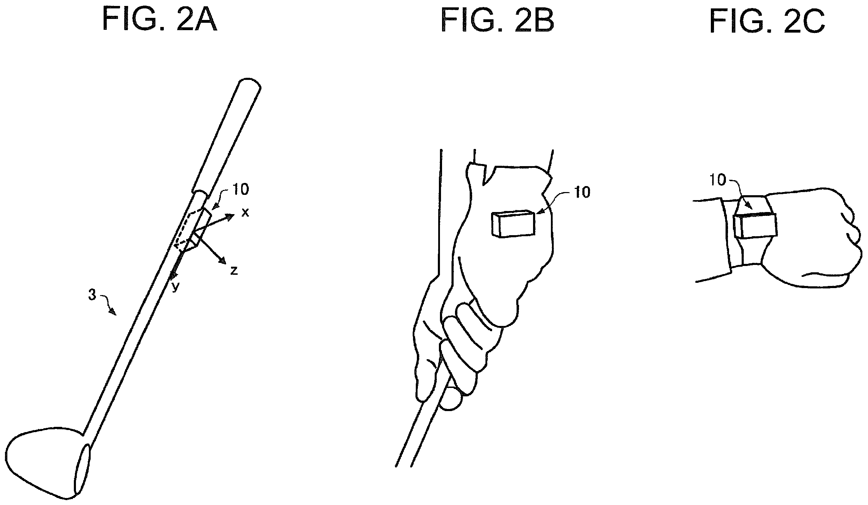

The sensor unit 10 may be attached to a part such as a shaft of the golf club 3 as illustrated in Fig. 2(A), may be attached to the hand or a glove of the user 2 as illustrated in Fig. 2(B), and may be attached to an accessory such as a wristwatch as illustrated in Fig. 2(C).

Particularly, as illustrated in Fig. 2 (A), if the sensor unit 10 is attached to the golf club 3 so that one axis of three detection axes (an x axis, a y axis, and a z axis), for example, the y axis matches a long axis direction of the shaft, a relative relationship between a direction of one detection axis of the sensor unit 10 and an attitude of the golf club 3 is fixed, and thus it is possible to reduce a computation amount in swing analysis. In a case where the sensor unit 10 is attached to the shaft of the golf club 3, as illustrated in Fig. 2(A), preferably, the sensor unit is attached to a position close to a grip portion to which impact during ball hitting is hardly forwarded and a centrifugal force is hardly applied.

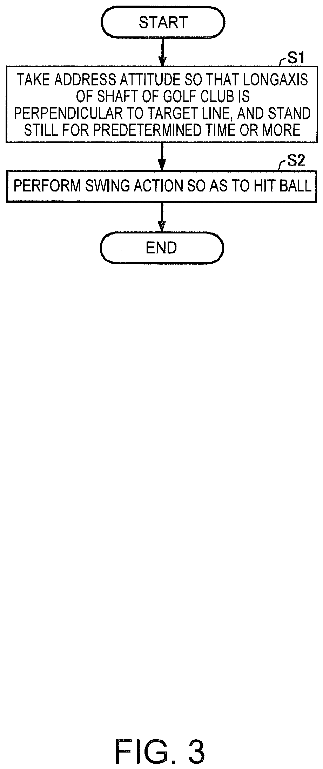

In the present embodiment, the user 2 performs a swing action for hitting a golf ball 4 according to predefined procedures. Fig. 3 is a diagram illustrating procedures of actions performed by the user 2. As illustrated in Fig. 3, first, the user 2 holds the golf club 3, takes an address attitude so that the long axis of the shaft of the golf club 3 is perpendicular to a target line (target hit ball direction), and stands still for a predetermined time period or more (for example, for one second or more) (step S1). Next, the user 2 performs a swing action so as to hit the golf ball 4 (step S2).

While the user 2 performs the action of hitting the golf ball 4 according to the procedures illustrated in Fig. 3, the sensor unit 10 measures three-axis accelerations and three-axis angular velocities in a predetermined cycle (for example, 1 ms), and sequentially transmits measured data to the motion analysis apparatus 20. Communication between the sensor unit 10 and the motion analysis apparatus 20 may be wireless communication, and may be wired communication.

The user 2 operates the motion analysis apparatus 20 before performing the actions illustrated in Fig. 3, so as to activate application software for swing analysis, and inputs information required in the analysis. The user 2 operates the motion analysis apparatus 20 so as to cause the sensor unit 10 to start measurement. The user 2 operates the motion analysis apparatus 20 so as to cause the sensor unit 10 to finish the measurement after performing the actions illustrated in Fig. 3. Then, the motion analysis apparatus 20 analyzes swing motion automatically or in response to the user's operation.

In a case where a specific state regarding the swing motion of the user 2 is detected by using the data measured by the sensor unit 10, the motion analysis apparatus 20 generates a control signal for controlling imaging performed by the imaging apparatus 30, and transmits the control signal to the imaging apparatus 30. The motion analysis apparatus 20 may detect a specific action in the swing motion in which the user 2 has hit the ball with the golf club 3, by using the data measured by the sensor unit 10. The motion analysis apparatus 20 may acquire image data captured by the imaging apparatus 30, and may generate analysis information in which the acquired image data is correlated with the specific action in the swing motion so as to present the analysis information to the user 2 by using an image or a sound. The motion analysis apparatus 20 may be, for example, a portable apparatus such as a smart phone, or a personal computer (PC).

The imaging apparatus 30 receives the control signal for starting imaging from the motion analysis apparatus 20, thus automatically starts capturing of moving images regarding the swing motion of the user 2 or continuous capturing of still images, and sequentially stores the captured images in a storage section built thereinto, during measurement in the sensor unit 10. The imaging apparatus 30 receives the control signal for finishing the imaging from the motion analysis apparatus 20 and thus automatically finishes the imaging. In other words, in the present embodiment, the user 2 can obtain images regarding the swing motion without operating the imaging apparatus 30.

1-1-2. Configuration of motion analysis system

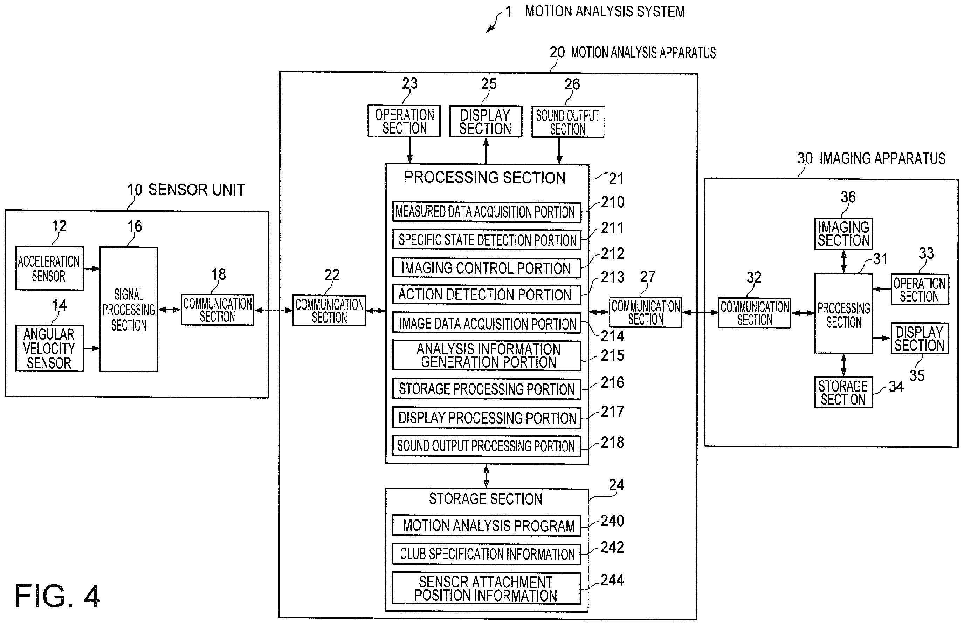

Fig. 4 is a diagram illustrating a configuration example of the motion analysis system 1 of the first embodiment. As illustrated in Fig. 4, the motion analysis system 1 of the first embodiment is configured to include the sensor unit 10, the motion analysis apparatus 20, and the imaging apparatus 30.

[Configuration of sensor unit]

As illustrated in Fig. 4, in the present embodiment, the sensor unit 10 is configured to include an acceleration sensor 12, an angular velocity sensor 14, a signal processing section 16, and a communication section 18.

The acceleration sensor 12 measures respective accelerations in three axial directions which intersect (ideally, orthogonal to) each other, and outputs digital signals (acceleration data) corresponding to magnitudes and directions of the measured three-axis accelerations.

The angular velocity sensor 14 measures respective angular velocities in three axial directions which intersect (ideally, orthogonal to) each other, and outputs digital signals (angular velocity data) corresponding to magnitudes and directions of the measured three-axis angular velocities.

The signal processing section 16 receives the acceleration data and the angular velocity data from the acceleration sensor 12 and the angular velocity sensor 14, respectively, adds measurement time points to the data, stores the data in a storage portion (not illustrated), generates packet data conforming to a communication format by using the stored measured data (the acceleration data and the angular velocity data), and outputs the packet data to the communication section 18.

Ideally, the acceleration sensor 12 and the angular velocity sensor 14 are provided in the sensor unit 10 so that the three axes thereof match three axes (an x axis, a y axis, and a z axis) of an orthogonal coordinate system (sensor coordinate system) defined for the sensor unit 10, but, actually, errors occur in installation angles. Therefore, the signal processing section 16 performs a process of converting the acceleration data and the angular velocity data into data in the xyz coordinate system by using a correction parameter which is calculated in advance according to the installation angle errors.

The signal processing section 16 may perform a process of correcting the temperatures of the acceleration sensor 12 and the angular velocity sensor 14. The acceleration sensor 12 and the angular velocity sensor 14 may have a temperature correction function.

The acceleration sensor 12 and the angular velocity sensor 14 may output analog signals, and, in this case, the signal processing section 16 may A/D convert an output signal from the acceleration sensor 12 and an output signal from the angular velocity sensor 14 so as to generate measured data (acceleration data and angular velocity data), and may generate communication packet data by using the data.

The communication section 18 performs a process of transmitting packet data received from the signal processing section 16 to the motion analysis apparatus 20, or a process of receiving a control signal (measurement control command) from the motion analysis apparatus 20 and sending the control command to the signal processing section 16. The signal processing section 16 performs various processes corresponding to measurement control commands. For example, if a measurement starting command is received, the signal processing section 16 causes the acceleration sensor 12 and the angular velocity sensor 14 to start measurement, and also starts generation of packet data. If a measurement finishing command is received, the signal processing section 16 causes the acceleration sensor 12 and the angular velocity sensor 14 to finish the measurement, and also finishes the generation of packet data.

[Configuration of motion analysis apparatus]

As illustrated in Fig. 4, in the present embodiment, the motion analysis apparatus 20 is configured to include a processing section 21, a communication section 22, an operation section 23, a storage section 24, a display section 25, a sound output section 26, and a communication section 27.

The communication section 22 performs a process of receiving packet data transmitted from the sensor unit 10 and sending the packet data to the processing section 21, or a process of receiving a control signal (measurement control command) for controlling measurement in the sensor unit 10 from the processing section 21 and transmitting the control signal to the sensor unit 10.

The operation section 23 performs a process of acquiring operation data from the user 2 or the like, and sending the operation data to the processing section 21. The operation section 23 may be, for example, a touch panel type display, a button, a key, or a microphone.

The storage section 24 is constituted of, for example, various IC memories such as a read only memory (ROM), a flash ROM, and a random access memory (RAM), or a recording medium such as a hard disk or a memory card.

The storage section 24 stores a program for the processing section 21 performing various computation processes or a control process, or various programs or data for realizing application functions. Particularly, in the present embodiment, the storage section 24 stores a motion analysis program 240 which is read by the processing section 21 and executes a motion analysis process. The motion analysis program 240 may be stored in a nonvolatile recording medium in advance, or the motion analysis program 240 may be received from a server by the processing section 21 via a network, and may be stored in the storage section 24.

In the present embodiment, the storage section 24 stores club specification information 242 indicating a specification of the golf club 3, and sensor attachment position information 244 indicating an attachment position of the sensor unit 10.

For example, the user 2 may operate the operation section 23 so as to sequentially input type numbers of the golf club 3 (alternatively, selects a type number from a type number list), and specification information of the input type number may be used as the club specification information 242 among specification information pieces (for example, information regarding a length of the shaft, a position of the centroid thereof, a lie angle, a face angle, a loft angle, and the like) for each type number is stored in the storage section 24 in advance. Alternatively, if the user 2 operates the operation section 23 so as to input a type number or the kind (a driver, or Nos. 1 to 9 iron clubs) of the golf club 3, the processing section 21 may display default values of various items such as a length of the shaft regarding a golf club of the input type number or kind on the display section 25 in an editable manner, and the club specification information 242 may include the default values or edited values of various items.

For example, the user 2 may input an attachment position of the sensor unit 10 and a distance to the grip of the golf club 3 by operating the operation section 23, and the input distance information may be stored in the storage section 24 as the sensor attachment position information 244. Alternatively, the sensor unit 10 may be attached at a defined predetermined position (for example, a distance of 20 cm from the grip), and thus information regarding the predetermined position may be stored as the sensor attachment position information 244 in advance.

The storage section 24 is used as a work area of the processing section 21, and temporarily stores data which is input from the operation section 23, results of calculation executed by the processing section 21 according to various programs, and the like. The storage section 24 may store data which is required to be preserved for a long period of time among data items generated through processing in the processing section 21.

The display section 25 displays a processing result in the processing section 21 as text, a graph, a table, animation, and other images. The display section 25 may be, for example, a CRT, an LCD, a touch panel type display, and a head mounted display (HMD). A single touch panel type display may realize functions of the operation section 23 and the display section 25.

The sound output section 26 displays a processing result in the processing section 21 as a sound such as a voice or a buzzer sound. The sound output section 26 may be, for example, a speaker or a buzzer.

The communication section 27 performs a process of receiving a control signal (imaging control command) for controlling imaging in the imaging apparatus 30 from the processing section 21 and transmitting the control signal to the imaging apparatus 30, or a process of receiving image data captured by the imaging apparatus 30 and imaging time points thereof and sending the image data and the imaging time points thereof to the processing section 21.

The processing section 21 performs a process of transmitting a measurement control command to the sensor unit 10, or performs various computation processes on data which is received via the communication section 22 from the sensor unit 10. The processing section 21 performs a process of transmitting an imaging control command to the imaging apparatus 30, or performs various processes on data which is received via the communication section 27 via the imaging apparatus 30. The processing section 21 performs other various control processes such as read/write processes of data for the storage section 24, a process of sending image data to the display section 25, and a process of sending sound data to the sound output section 26, according to operation data received from the operation section 23. Particularly, in the present embodiment, by executing the motion analysis program 240, the processing section 21 functions as a measured data acquisition portion 210, a specific state detection portion 211, an imaging control portion 212, an action detection portion 213 , an image data acquisition portion 214, an analysis information generation portion 215, a storage processing portion 216, a display processing portion 217, and a sound output processing portion 218.

The measured data acquisition portion 210 performs a process of receiving packet data which is received from the sensor unit 10 by the communication section 22, and acquiring measurement time points and measured data from the received packet data. The measurement time points and the measured data acquired by the measured data acquisition portion 210 are stored in the storage section 24 in correlation with each other.

The specific state detection portion 211 performs a process of detecting specific states regarding a swing of the user 2 by using the measured data output from the sensor unit 10. In the present embodiment, the specific state detection portion 211 detects a first state as one of the specific states. The first state is, for example, a standing still state before the user 2 starts a swing (a standing still state at address). The specific state detection portion 211 detects a second state as one of the specific states. The second state is, for example, a standing still state after the user 2 finishes a swing (a standing still state after follow-through).

In a case where the specific state detection portion 211 detects the specific state, the imaging control portion 212 performs a process of generating a control signal (imaging control command) for causing the imaging apparatus 30 to control imaging, and transmitting the imaging control command to the imaging apparatus 30 via the communication section 27. In the present embodiment, in a case where the specific state detection portion 211 detects the first state (a standing still state before the user 2 starts a swing), the imaging control portion 212 generates a first control signal (imaging starting command) for causing the imaging apparatus 30 to start imaging, and transmits the first control signal to the imaging apparatus 30. Consequently, measured data (an output signal from the sensor unit 10) output from the sensor unit 10 is synchronized with image data captured by the imaging apparatus 30 on the basis of a timing of the first state.

In the present embodiment, in a case where the specific state detection portion 211 detects the second state (a standing still state after the user 2 finishes a swing), the imaging control portion 212 generates a second control signal (imaging finishing command) for causing the imaging apparatus 30 to finish imaging, and transmits the second control signal to the imaging apparatus 30.

The action detection portion 213 performs a process of detecting an action in a swing of the user 2, and specifying a detection time point (a measurement time point of the measured data), by using the measured data output from the sensor unit 10. In the present embodiment, the action detection portion 213 detects a plurality of characteristic actions in a swing. For example, the action detection portion 213 detects an action when the user 2 starts a swing (for example, an action right after starting a backswing). The action detection portion 213 detects an action when the user 2 switches a swing direction (for example, a top at which the swing changes from the backswing to a downswing). The action detection portion 213 detects an action (an action (natural uncock) of lessening force of the wrists during a downswing of the user 2) when a swing speed becomes the maximum. The action detection portion 213 detects an action (for example, impact) when the user 2 hits the ball. The action detection portion 213 detects an action (for example, an action right before finishing follow-through) when the user 2 finishes a swing.

Specifically, first, the action detection portion 213 computes an offset amount included in the measured data by using the measured data (the acceleration data and the angular velocity data) at during standing still (at address) of the user 2, stored in the storage section 24, after the sensor unit 10 finishes the measurement. Next, the action detection portion 213 performs bias correction on the measured data by subtracting the offset amount from the measured data stored in the storage section 24, and detects each characteristic according to in a swing of the user 2 by using the measured data having undergone the bias correction. For example, the action detection portion 213 may compute a combined value of the acceleration data or the angular velocity data having undergone the bias correction, and may detect respective actions right after a backswing is started, at the top, at impact, and right before follow-through is finished, on the basis of the combined value. For example, the action detection portion 213 may compute a grip speed by using an integral value of the acceleration data having undergone the bias correction, and the club specification information 242 and the sensor attachment position information 244, and may detect the time at which the grip speed is the maximum, as the natural uncock.

The image data acquisition portion 214 performs a process of acquiring image data captured by the imaging apparatus 30 and imaging time points via the communication section 27. The image data and the imaging time points acquired by the image data acquisition portion 214 are stored in the storage section 24 in correlation with each other.

The analysis information generation portion 215 performs a process of correlating the image data acquired by the image data acquisition portion 214 with the action detected by the action detection portion 213. For example, after the imaging control portion 212 transmits an imaging starting command, the analysis information generation portion 215 may convert an imaging time point of each image data item into a measurement time point by using a measurement time point of the latest measured data acquired by the measured data acquisition portion as an imaging starting time point in the imaging apparatus 30, and may correlates each action detected by the action detection portion 213 with each image data item in which the converted imaging time point matches (or close to) a measurement time point at which the action is detected. In the present embodiment, among image data items acquired by the image data acquisition portion 214, the analysis information generation portion 215 attaches flags of different kinds to image data items corresponding to respective actions detected by the action detection portion 213 according to the kind of the detected action. For example, the analysis information generation portion 215 attaches a flag 1 (first flag) to image data corresponding to the action when the user 2 starts a swing. The analysis information generation portion 215 attaches a flag 2 (second flag) to image data corresponding to the action when the user 2 switches a direction of the swing. The analysis information generation portion 215 attaches a flag 3 (third flag) to image data corresponding to the action when a swing speed is the maximum. The analysis information generation portion 215 attaches a flag 4 (fourth flag) to image data corresponding to the action when the user 2 hits the ball. The analysis information generation portion 215 attaches a flag 5 (fifth flag) to image data corresponding to the action when the user 2 finishes the swing.

The analysis information generation portion 215 generates analysis information including a correspondence relationship between the image data acquired by the image data acquisition portion 214 and the action detected by the action detection portion 213. For example, the analysis information generation portion 215 generates analysis information in which text representing each characteristic action (actions such as the top, the natural uncock, and the impact) in a swing is correlated with image data (a captured image of each action) corresponding to each action.

The analysis information generation portion 215 may define an XYZ coordinate system (global coordinate system) which has a target line indicating a target hit ball direction as an X axis, an axis on a horizontal plane which is perpendicular to the X axis as a Y axis, and a vertically upward direction (a direction opposite to the gravitational direction) as a Z axis, and may compute a position and an attitude of the sensor unit 10 in the XYZ coordinate system (global coordinate system) by using measured data which is subjected to bias correction as a result of subtracting an offset amount from the measured data. For example, the analysis information generation portion 215 may compute changes in positions change from an initial position of the sensor unit 10 in a time series by performing second order differentiation on acceleration data, and may compute changes in attitudes from an initial attitude of the sensor unit 10 in a time series by performing rotation calculation using angular velocity data. An attitude of the sensor unit 10 may be expressed by, for example, rotation angles (a roll angle, a pitch angle, and a yaw angle) about the X axis, the Y axis, and the Z axis, Euler angles, or a quaternion.

Since the user 2 performs the action in step S1 in Fig. 3, the initial position of the sensor unit 10 has an X coordinate of 0. Since the acceleration sensor 12 measures only the gravitational acceleration during standing still of the user 2, for example, as illustrated in Fig. 2(A), in a case where the y axis of the sensor unit 10 matches the long axis direction of the shaft of the golf club 3, the analysis information generation portion 215 may compute an inclined angle (an inclination relative to a horizontal plane (XY plane) or a vertical plane (XZ plane)) of the shaft by using y axis acceleration data. The analysis information generation portion 215 may compute a Y coordinate and a Z coordinate of the initial position of the sensor unit 10 by using the inclined angle of the shaft, the club specification information 242 (the length of the shaft), and the sensor attachment position information 244, so as to specify the initial position of the sensor unit 10.

Since the acceleration sensor 12 measures only the gravitational acceleration during standing still of the user 2, the analysis information generation portion 215 may specify an angle formed between each of the x axis, the y axis, and the z axis of the sensor unit 10, and the gravitational direction by using three-axis acceleration data. Since the user 2 performs the action in step S1 in Fig. 3, and thus the y axis of the sensor unit 10 is present on the YZ plane during standing still of the user 2, the analysis information generation portion 215 can specify the initial attitude of the sensor unit 10.

The analysis information generation portion 215 may compute a trajectory of the golf club 3 in a swing by using the position information and the attitude information of the sensor unit 10, and may generate analysis information for causing the trajectory of the golf club 3 and images (moving images or continuously captured still images) captured by the imaging apparatus 30 to be displayed in an overlapping manner, on the basis of the correspondence relationship between the image data and the characteristic action.

The analysis information generation portion 215 may generate analysis information including a head speed during hitting of the ball, an incidence angle (club path) or a face angle during hitting of the ball, shaft rotation (a change amount of a face angle during a swing), and a deceleration rate of the golf club 3, or information regarding a variation in these information pieces in a case where the user 2 performs a plurality of swings, by using the position information the attitude information of the sensor unit 10.

The signal processing section 16 of the sensor unit 10 may compute an offset amount of measured data so as to perform bias correction on the measured data, and the acceleration sensor 12 and the angular velocity sensor 14 may have a bias correction function. In this case, it is not necessary for the action detection portion 213 or the analysis information generation portion 215 to perform bias correction on the measured data.

The storage processing portion 216 performs read/write processes of various programs or various data for the storage section 24. Specifically, the storage processing portion 216 performs a process of storing the measured data acquired by the measured data acquisition portion 210 in the storage section 24 in correlation with measurement time points, or a process of reading the information from the storage section 24. The storage processing portion 216 performs a process of storing the image data acquired by the image data acquisition portion 214 in the storage section 24 in correlation with imaging time points, or a process of reading the information from the storage section 24. The storage processing portion 216 also performs a process of storing the club specification information 242 and the sensor attachment position information 244 corresponding to information which is input by the user 2 operating the operation section 23, in the storage section 24, or a process of reading the information from the storage section 24. The storage processing portion 216 also performs a process of storing information regarding a measurement time point at which the imaging control portion 212 transmits an imaging starting command or an imaging finishing command, information for specifying each action detected by the action detection portion 213, the analysis information generated by the analysis information generation portion 215, and the like, in the storage section 24, or a process of reading the information from the storage section 24.

The display processing portion 217 performs a process of displaying various images (including text, symbols, and the like) on the display section 25. For example, the display processing portion 217 performs a process of generating an image corresponding to the analysis information stored in the storage section 24 automatically or in response to an input operation of the user 2 after swing motion of the user 2 is finished, and displaying the image on the display section 25. A display section may be provided in the sensor unit 10, and the display processing portion 217 may transmit various image data items to the sensor unit 10 via the communication section 22, and various images may be displayed on the display section of the sensor unit 10.

The sound output processing portion 218 performs a process of outputting various sounds (including voices, buzzer sounds, and the like) from the sound output section 26. For example, the sound output processing portion 218 may generate a sound or a voice corresponding to the analysis information stored in the storage section 24 automatically or in response to an input operation of the user 2 after swing motion of the user 2 is finished, and may output the sound or the voice from the sound output section 26. A sound output section may be provided in the sensor unit 10, and the sound output processing portion 218 may transmit various sound data items or voice data items to the sensor unit 10 via the communication section 22 and may output various sounds or voices from the sound output section of the sensor unit 10.

A vibration mechanism may be provided in the motion analysis apparatus 20 or the sensor unit 10, and various pieces of information may be converted into vibration information by the vibration mechanism so as to be presented to the user 2.

[Configuration of imaging apparatus]

As illustrated in Fig. 4, in the present embodiment, the imaging apparatus 30 is configured to include a processing section 31, a communication section 32, an operation section 33, a storage section 34, a display section 35, and an imaging section 36.

The communication section 32 performs a process of receiving captured image data and information regarding imaging time points thereof from the processing section 31 and transmitting the data and the information to the motion analysis apparatus 20, or a process of receiving an imaging control command from the motion analysis apparatus 20 and sending the imaging control command to the processing section 31.

The operation section 33 performs a process of acquiring operation data from the user 2 or the like and sending the operation data to the processing section 31. The operation section 33 may be, for example, a touch panel type display, a button, a key, or a microphone.

The imaging section 36 performs a process of generating image data of moving images or still images corresponding to light emitted from a subject (user 2), and sending the generated image data to the processing section 31. For example, the imaging section 36 receives light emitted from the subject (user 2) with an imaging element (not illustrated) through a lens (not illustrated), converts the light into an electric signal, decomposes the electric signal into RGB components, and performs desired adjustment or correction and A/D conversion so as to generate image data.

If an instruction for capturing a still image is received from the processing section 31, the imaging section 36 generates image data of the still image. If an instruction for starting capturing of a moving image is received from the processing section 31, the imaging section 36 generates image data of the moving image at a set frame rate (for example, 60 frames/second). If an instruction for starting continuous capturing of still images is received from the processing section 31, the imaging section 36 continuously generates image data of the still images at a set time interval (for example, an interval of 0.1 seconds). If an instruction for finishing imaging is received from the processing section 31, generation of image data is finished.

The storage section 34 is constituted of, for example, various IC memories such as a ROM, a flash ROM, and a RAM, or a recording medium such as a hard disk or a memory card.

The storage section 34 stores a program or data for the processing section 31 performing various computation processes or a control process. The storage section 34 is used as a work area of the processing section 31, and temporarily stores data which is input from the operation section 33, results of calculation executed by the processing section 31 according to various programs, and the like. The recording medium included in the storage section 34 stores data (image data ortl) which is required to be preserved for a long period of time.

The processing section 31 performs a process of receiving a imaging control command which is received from the motion analysis apparatus 20 by the communication section 32, and controlling the imaging section 36 in response to the received imaging control command. Specifically, in a case where an imaging starting command is received, the processing section 31 instructs the imaging section 36 to start capturing of moving images or to start continuous capturing of still images. Whether the processing section 31 instructs capturing of moving images and continuous capturing of still images to be started may be set in advance, and may be selected by the user 2 or the like. If an imaging finishing command is received, the processing section 31 instructs the imaging section 36 to finish the imaging.

In response to information which is input by the user 2 or the like via the operation section 33, the processing section 31 instructs the imaging section 36 to capture still images, to start capturing of moving images, to start continuous capturing of still images, and to finish the imaging.

The processing section 31 performs a process of receiving image data from the imaging section 36, adding imaging time points to the image data, storing the image data in the storage section 34, and sending the image data to the display section 35. The processing section 31 performs a process of selecting image data corresponding to a selection operation of the user 2 or the like from among the image data stored in the storage section 34, and sending the image data to the display section 35.

The processing section 31 performs a process of reading image data of the latest moving images or still images (continuously captured still images) which are captured and stored in the storage section 34 along with information regarding imaging time points at a desired timing after the imaging finishing command is received, and transmitting the data and the information to the motion analysis apparatus 20 via the communication section 32.

The processing section 31 performs other various control processes such as read/write processes of data for the storage section 34, and a process of sending image data to the display section 35, according to the operation data received from the operation section 33.

The display section 35 receives image data from the processing section 31 and displays an image corresponding to the image data. The display section 35 may be, for example, a CRT, an LCD, or a touch panel type display. A single touch panel type display may realize the functions of the operation section 33 and the display section 35.

The imaging apparatus 30 may include a sound collection section (a microphone or the like) which acquires sounds during imaging, or a sound output section (a speaker or the like) which outputs the acquired sounds along with reproduction of moving images. The imaging apparatus 30 may have a function of communicating with other apparatuses through connection to the Internet or a LAN.

1-1-3. Imaging control

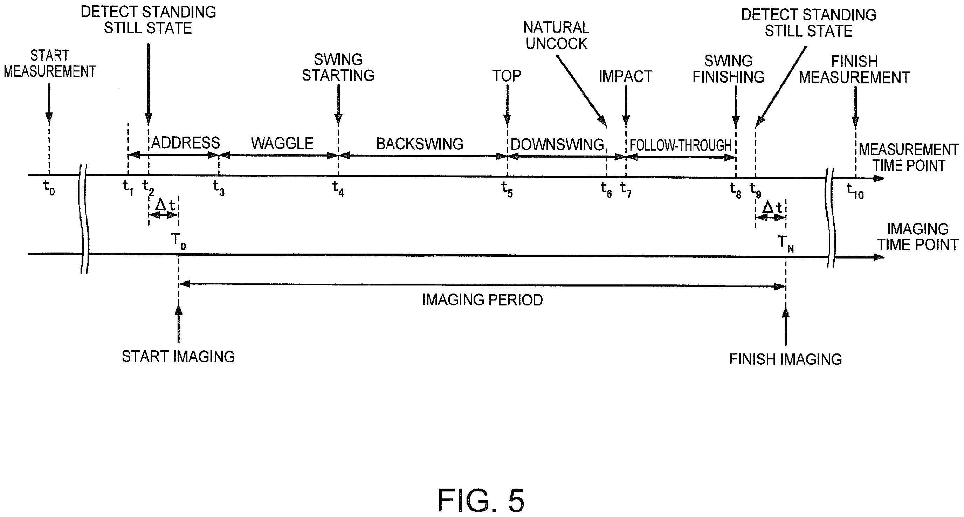

Fig. 5 is a diagram illustrating an example of imaging control performed by the processing section 21. In the example illustrated in Fig. 5, a measurement time point of initial measured data after the sensor unit 10 starts measurement is set to $t_0$. Then, the user 2 stands still at an address attitude from a measurement time point $t_1$ to a measurement time point $t_3$ (step S1 in Fig. 3), and the processing section 21 detects a standing still state before the user 2 starts a swing and causes the imaging apparatus 30 to start imaging at the measurement time point $t_2$. Here, if an imaging time point at which the imaging apparatus 30 starts imaging is set to $T_0$, and a delay time until the imaging apparatus 30 starts the imaging after the processing section 21 acquires measured data at the measurement time point $t_2$ is set to $\Delta t$, the imaging time point $T_0$ corresponds to the measurement time point $t_2+\Delta t$.

Then, the user 2 performs an action of slightly moving the hands and the feet, called waggling, from a measurement time point $t_3$ to a measurement time point $t_4$, and starts a swing at the measurement time point $t_4$. A period from the measurement time point $t_4$ to a measurement time point $t_5$ is a backswing period, and a period from the measurement time point $t_5$ to a measurement time point $t_7$ is a downswing period. A top occurs in the swing at the measurement time point $t_5$ at which the swing switches from the backswing to the downswing, and impact occurs at the measurement time point $t_7$. Natural uncock occurs at the measurement time point $t_6$ slightly before the impact.

A period from a measurement time point $t_7$ to a measurement time point $t_8$ is a follow-through period, and the swing is finished at the measurement time point $t_8$ at which the follow-through is completed. Next, the processing section 21 detects a standing still state after the user 2 finishes the swing at a measurement time point $t_9$, and causes the imaging apparatus 30 to finish the imaging. Here, if an imaging time point at which the imaging apparatus 30 finishes the imaging is set to $T_N$, and a delay time until the imaging apparatus 30 finishes the imaging after the processing section 21 acquires measured data at the measurement time point $t_9$ is set to $\Delta t$, the imaging time point $T_N$ corresponds to the measurement time point $t_9+\Delta t$.

Next, if the sensor unit 10 finishes the measurement at a measurement time point $t_{10}$, the processing section 21 detects respective actions at the swing starting, the top, the natural uncock, the impact, and the swing finishing by using measured data at the measurement time points $t_1$ to $t_9$, and specifies the measurement time point $t_4$, $t_5$, $t_6$, $t_7$ and $t_8$ corresponding to the actions.

The processing section 21 acquires image data captured in the imaging period at the imaging time points $T_0$ to $T_N$ from the imaging apparatus 30, and correlates each detected action with the image data. Specifically, the processing section 21 adds flags 1 to 5 to the image data items corresponding to the respective detected actions.

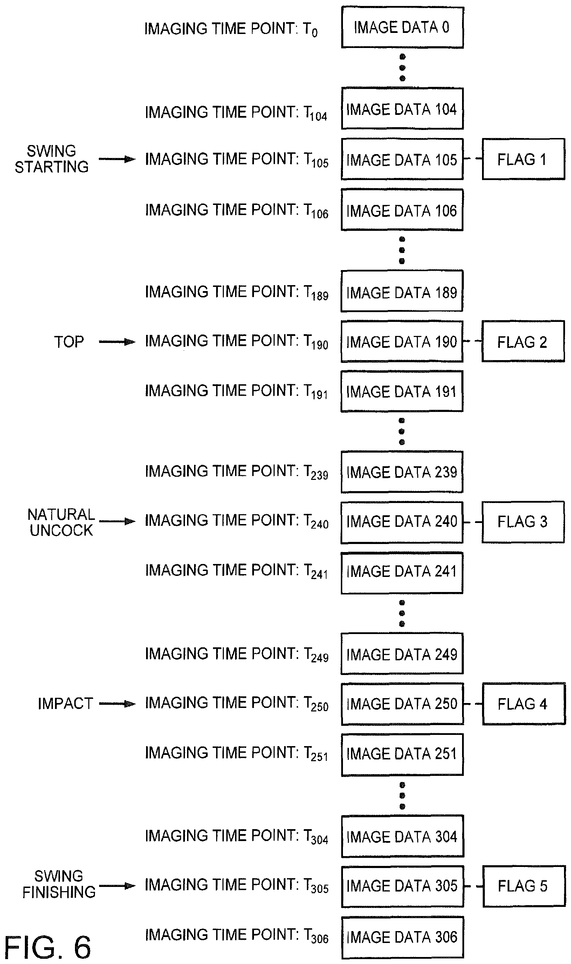

Fig. 6 is a diagram illustrating a correspondence relationship between the image data and each action in the example illustrated in Fig. 5. As illustrated in Fig. 6, the flag 1 is added to image data 105 at the imaging time point $T_{105}$ corresponding to the measurement time point $t_4$ at which the swing is started. For example, $T_{105}$ is $T_0+(t_4-t_2-\Delta t)$. The flag 2 is added to image data 190 at the imaging time point $T_{190}$ corresponding to the measurement time point $t_5$ at the top. For example, $T_{190}$ is $T_0+(t_5-t_2-\Delta t)$. The flag 3 is added to image data 240 at the imaging time point $T_{240}$ corresponding to the measurement time point $t_6$ at the natural uncock. For example, $T_{240}$ is $T_0+(t_6-t_2-\Delta t)$. The flag 4 is added to image data 250 at the imaging time point $T_{250}$ corresponding to the measurement time point $t_7$ at the impact. For example, $T_{250}$ is $T_0+(t_7-t_2-\Delta t)$. The flag 5 is added to image data 305 at the imaging time point $T_{305}$ corresponding to the measurement time point $t_8$ at which the swing is finished. For example, $T_{305}$ is $T_0+(t_8-t_2-\Delta t)$.

As mentioned above, since respective images corresponding to the characteristic actions in a swing are added with the flags of different types so as to be displayed, the user 2 can easily find an image corresponding to each action, and can thus easily perform image editing work.

1-1-4. Process in motion analysis apparatus

[Motion analysis process]

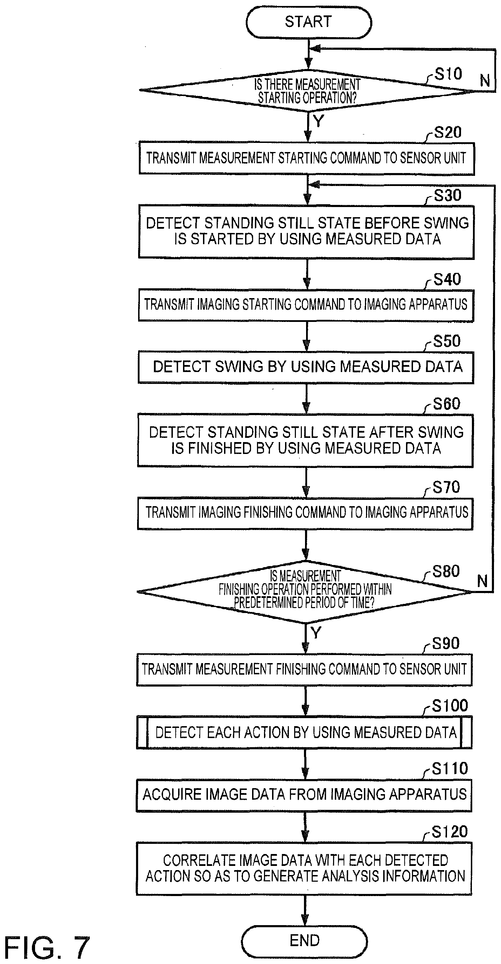

Fig. 7 is a flowchart illustrating examples (examples of a motion analysis method) of procedures of a motion analysis process performed by the processing section 21 of the motion analysis apparatus 20 in the first embodiment. The processing section 21 of the motion analysis apparatus 20 (an example of a computer) performs the motion analysis process, for example, according to the procedures shown in the flowchart of Fig. 7 by executing the motion analysis program 240 stored in the storage section 24. Hereinafter, the flowchart of Fig. 7 will be described.

First, the processing section 21 determines whether or not a measurement starting operation has been performed on the basis of operation data (step S10), and waits for the measurement starting operation to be performed (N in step S10). In a case where the measurement starting operation has been performed (Y in step S10), the processing section 21 transmits a measurement starting command to the sensor unit 10 (step S20). The sensor unit 10 receives the measurement starting command, and starts to measure three-axis accelerations and three-axis angular velocities. Next, the processing section 21 sequentially acquires measured data which is output from the sensor unit 10, and stores the measured data in the storage section 24. The user 2 performs the actions in steps S1 and S2 in Fig. 3.

Next, the processing section 21 detects a standing still state (a standing still state at address) before the user 2 starts a swing, by using the measured data output from the sensor unit 10 (step S30). For example, the processing section 21 detects the standing still state in a case where a combined value of three-axis accelerations having undergone bias correction or three-axis angular velocities having undergone bias correction is continuously equal to or smaller than a predetermined threshold value for a predetermined period of time.

Next, the processing section 21 transmits an imaging starting command to the imaging apparatus 30 (step S40). The imaging apparatus 30 receives the imaging starting command, and starts imaging.

Next, the processing section 21 detects the swing by using the measured data output from the sensor unit 10 (step S50). For example, the processing section 21 detects the swing in a case where the combined value of three-axis accelerations having undergone bias correction or three-axis angular velocities having undergone bias correction exceeds the predetermined threshold value (for example, during a downswing or at impact).

Next, the processing section 21 detects a standing still state after the user 2 finishes the swing by using the measured data output from the sensor unit 10 (step S60). For example, the processing section 21 detects the standing still state in a case where a combined value of three-axis accelerations having undergone bias correction or three-axis angular velocities having undergone bias correction is continuously equal to or smaller than a predetermined threshold value for a predetermined period of time. The detection process in step S50 is provided so that the standing still state before performing the swing is not erroneously detected in the detection process in step S60.

Next, the processing section 21 transmits an imaging finishing command to the imaging apparatus 30 (step S70). The imaging apparatus 30 receives the imaging finishing command, and finishes the imaging.

Next, the processing section 21 determines whether or not a measurement finishing operation has been performed within a predetermined period of time on the basis of operation data (step S80), and performs the processes in step S30 and the subsequent steps again in a case where the measurement finishing operation has not been performed within the predetermined period of time (N in step S80). The user 2 performs the actions in steps S1 and S2 in Fig. 3.

In a case where the measurement finishing operation has been performed within the predetermined period of time (Y in step S80), the processing section 21 transmits a measurement finishing command to the sensor unit 10 (step S90). The sensor unit 10 receives the measurement finishing command, and finishes the measurement of three-axis accelerations and three-axis angular velocities.

Next, the processing section 21 detects each characteristic action in the swing by using the measured data which stored in the storage section 24 after step S30 (step S100). Specific procedures of the process in step S100 will be described later.

Next, the processing section 21 acquires captured image data from the imaging apparatus 30 (step S110).

The processing section 21 correlates the image data acquired in step S110 with each action detected in step S100 so as to generate analysis information (step S120), and finishes the process.

In the flowchart of Fig. 7, order of the respective steps may be changed as appropriate within an allowable range.

[Action detection process]

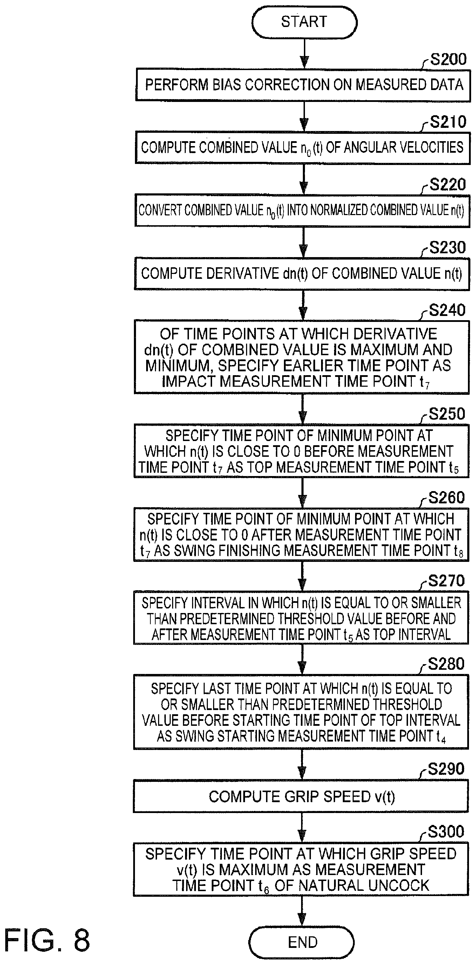

Fig. 8 is a flowchart illustrating examples of procedures of a process (the process in step S100 in Fig. 7) of detecting each action in the swing of the user 2. Hereinafter, the flowchart of Fig. 8 will be described.

First, the processing section 21 performs bias correction on the measured data (the acceleration data and the angular velocity data) stored in the storage section 24 (step S200).

Next, the processing section 21 computes a combined value $n_0(t)$ of angular velocities at each time point t by using the angular velocity data (angular velocity data for each time point t) having undergone the bias correction in step S200 (step S210). For example, if the angular velocity data items at the time point t are respectively indicated by x(t), y(t), and z(t), the combined value $n_0(t)$ of the angular velocities is computed according to the following Equation (1).

[Expression 1]

$$n_0(t) = \sqrt{x(t)^2 + y(t)^2 + z(t)^2} \cdots (1)$$

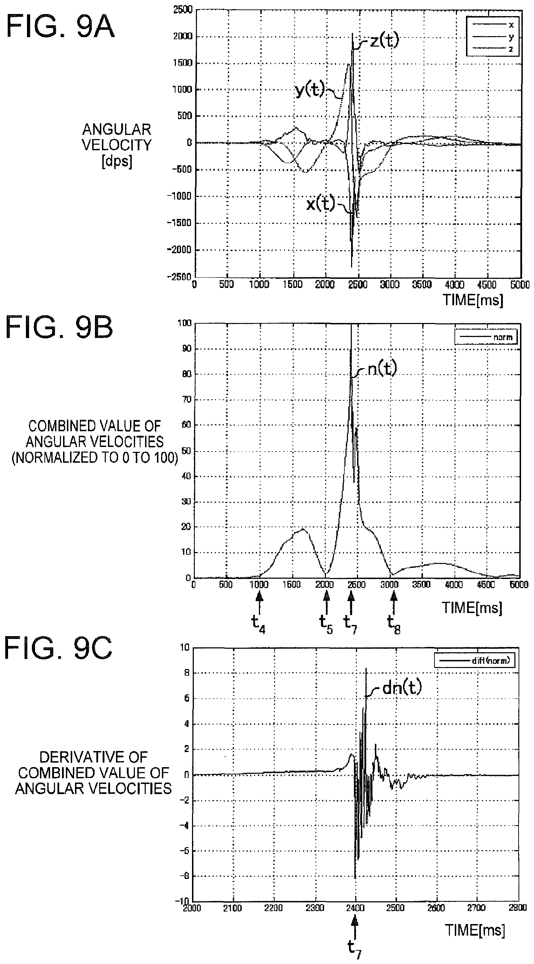

Fig. 9(A) illustrates examples the three-axis angular velocity data items x(t), y(t) and z(t) when the user 2 hits the golf ball 4 by performing the swing. In Fig. 9(A), a transverse axis expresses time (msec), and a longitudinal axis expresses angular velocity (dps).

Next, the processing section 21 converts the combined value $n_0(t)$ of the angular velocities at each time point t into a combined value n(t) which is normalized (scale-conversion) within a predetermined range (step S220). For example, if the maximum value of the combined value of the angular velocities in an acquisition period of measured data is $max(n_0)$, the combined value $n_0(t)$ of the angular velocities is converted into the combined value n(t) which is normalized within a range of 0 to 100 according to the following Equation (2).

[Expression 2]

$$n(t) = \frac{100 \times n_0(t)}{max(n_0)} \cdots (2)$$

Fig. 9(B) is a diagram in which the combined value $n_0(t)$ of the three-axis angular velocities is calculated according to Equation (1) by using the three-axis angular velocity data items x(t), y(t) and z(t) in Fig. 9(A), and then the combined value n(t) normalized to 0 to 100 according to Equation (2) is displayed in a graph. In Fig. 9(B), a transverse axis expresses time (msec), and a longitudinal axis expresses a combined value of the angular velocity.

Next, the processing section 21 computes a derivative dn(t) of the normalized combined value n(t) at each time point t (step S230). For example, if a cycle for measuring three-axis angular velocity data items is indicated by $\Delta t$, the derivative (difference) dn(t) of the combined value of the angular velocities at the time point t is calculated by using the following Equation (3).

[Expression 3]

$$dn(t) = n(t) - n(t - \Delta t) \cdots (3)$$

Fig. 9(C) is a diagram in which the derivative dn(t) is calculated according to Equation (3) on the basis of the combined value n(t) of the three-axis angular velocities in Fig. 9(B), and is displayed in a graph. In Fig. 9(C), a transverse axis expresses time (msec), and a longitudinal axis expresses a derivative value of the combined value of the three-axis angular velocities. In Fig. 9(A) and Fig. 9(B), the transverse axis is displayed at 0 to 5 seconds, but, in Fig. 9(C), the transverse axis is display at 2 to 2.8 seconds so that changes in the derivative value before and after ball hitting can be understood.

Next, of time points at which a value of the derivative dn (t) of the combined value becomes the maximum and the minimum, the processing section 21 detects the earlier time point as the impact measurement time point $t_7$ (step S240) (refer to Fig. 9 (C)). It is considered that a swing speed is the maximum at the moment of impact in a typical golf swing. Since it is considered that a value of the combined value of the angular velocities also changes according to a swing speed, a timing at which a derivative value of the combined value of the angular velocities is the maximum or the minimum (that is, a timing at which the derivative value of the combined value of the angular velocities is a positive maximum value or a negative minimum value) in a series of swing actions can be captured as a timing of impact. Since the golf club 3 vibrates due to the impact, a timing at which a derivative value of the combined value of the angular velocities is the maximum and a timing at which a derivative value of the combined value of the angular velocities is the minimum may occur in pairs, and, of the two timings, the earlier timing may be the moment of the impact.

Next, the processing section 21 specifies a time point of a minimum point at which the combined value n(t) is close to 0 before the impact measurement time point $t_7$, as the top measurement time point $t_5$ (step S250) (refer to Fig. 9(B)). It is considered that, in a typical golf swing, an action temporarily stops at the top after starting the swing, then a swing speed increases, and finally impact occurs. Therefore, a timing at which the combined value of the angular velocities is close to 0 and becomes the minimum before the impact timing may be captured as the top timing.

Next, the processing section 21 specifies a time point of a minimum point at which the combined value n(t) is close to 0 after the impact measurement time point $t_7$, as the swing finishing measurement time point $t_8$ (step S260) (refer to Fig. 9 (B)). It is considered that, in a typical golf swing, a swing speed gradually decreases after impact, and then the swing stops. Therefore, a timing at which the combined value of the angular velocities is close to 0 and becomes the minimum after the impact timing may be captured as the swing finishing timing.

Next, the processing section 21 specifies an interval in which the combined value n(t) is equal to or smaller than a predetermined threshold value before and after the top measurement time point $t_5$, as a top interval (step S270). It is considered that, in a typical golf swing, an action temporarily stops at the top, and thus a swing speed is low before and after the top. Therefore, an interval in which the combined value of angular velocities is continuously equal to or smaller than the predetermined threshold value along with the top timing may be specified as the top interval.

Next, the processing section 21 specifies a last time point at which the combined value n(t) is equal to or smaller than the predetermined threshold value before a starting time point of the top interval, as the swing starting measurement time point $t_4$ (step S280) (refer to Fig. 9 (B)). It is hardly considered that, in a typical golf swing, a swing action is started from a standing still state, and the swing action is stopped till the top. Therefore, the last timing at which the combined value of the angular velocities is equal to or smaller than the predetermined threshold value before the top interval may be captured as a timing of starting the swing action. A time point of the minimum point at which the combined value n(t) is close to 0 before the top measurement time point $t_5$ may be specified as the swing starting measurement time point.

Next, the processing section 21 computes a grip speed v(t) at each time point t by using the acceleration data (acceleration data at each time point t) having undergone the bias correction in step S200 (step S290).

Finally, the processing section 21 specifies a time point at which the grip speed v(t) is the maximum, as the natural uncock measurement time point $t_6$ (step S300), and finishes the process.

In the flowchart of Fig. 8, order of the respective steps may be changed as appropriate within an allowable range. In the flowchart of Fig. 8, the processing section 21 specifies the impact and the like by using the three-axis angular velocity data, but may similarly specify the impact and the like by using the three-axis acceleration data.

1-1-5. Effects

As described above, in the motion analysis system 1 of the first embodiment, the motion analysis apparatus 20 detects each characteristic action in a swing of the user 2 by using measured data output from the sensor unit 10, and correlates image data captured by the imaging apparatus 30 with each detected action, and thus the user 2 can specify the captured image data in correlation with each characteristic action. Therefore, it is possible to reduce time and effort to perform work of editing captured images.

Particularly, since the motion analysis apparatus 20 detects the actions such as the swing starting, the top, the natural uncock, the impact, and the swing finishing, and adds the flags 1 to 5 of different types to image data items corresponding to the respective detected actions, it is possible to easily specify the captured image data in correlation with each characteristic action. Therefore, it is possible to considerably reduce time and effort to perform work of editing captured images.

As described above, in the motion analysis system 1 of the first embodiment, in a case where the motion analysis apparatus 20 detects a specific state regarding a swing of the user 2 by using measured data output from the sensor unit 10, the imaging control command for controlling imaging is transmitted to the imaging apparatus 30, and thus imaging performed by the imaging apparatus 30 can be automatically controlled in conjunction with swing motion of the user 2.

For example, since the motion analysis apparatus 20 transmits the imaging starting command to the imaging apparatus 30 in a case of detecting a standing still state (address) before a swing is started, and transmits the imaging finishing command to the imaging apparatus 30 in a case of detecting a standing still state after the swing is finished, it is possible to automatically image the moment of a characteristic action such as the top, the natural uncock, or the impact in the swing without the user 2 performing an imaging starting or finishing operation on the imaging apparatus 30, and also to considerably reduce a data amount of captured images.

For example, the motion analysis apparatus 20 transmits a high-resolution setting command to the imaging apparatus 30 in a case detecting a standing still state (address) before a swing is started, and transmits a low-resolution setting command to the imaging apparatus 30 in a case of detecting a standing still state after the swing is finished. Therefore, the user 2 can image the moment of a characteristic action in the swing at a high resolution without performing an operation of changing the resolution, and can also continue to perform imaging while reducing a data amount before address or after swing finishing.

In the motion analysis system 1 of the first embodiment, the motion analysis apparatus 20 may cause the imaging apparatus 30 to capture moving images or to continuously capture still images. If the imaging apparatus 30 is caused to capture moving images, the user 2 can view moving images of a swing. On the other hand, if the imaging apparatus 30 is caused to continuously capture still images, the user 2 can view frame advance images along with high quality images of the characteristic action in the swing.

In the motion analysis system 1 of the first embodiment, the motion analysis apparatus 20 analyzes a swing of the user 2 by using the measured data output from the sensor unit 10, and thus there is less restriction in a location where swing analysis is performed compared with a case where a swing of the user 2 is analyzed by analyzing images captured from a plurality of directions.

1-2. Second embodiment

1-2-1. Summary of motion analysis system

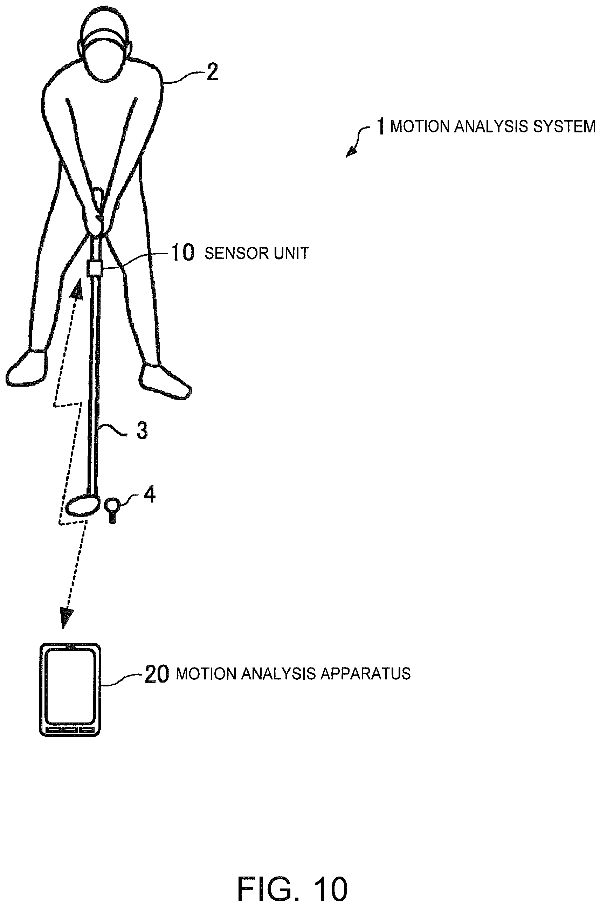

In a motion analysis system of the second embodiment, the same constituent elements as those in the first embodiment are given the same reference numerals, and description overlapping the first embodiment will be made briefly or be omitted. Fig. 10 is a diagram for explaining a summary of a motion analysis system according to the second embodiment. As illustrated in Fig. 10, a motion analysis system 1 of the second embodiment is configured to include a sensor unit 10 and a motion analysis apparatus 20.

The sensor unit 10 can measure three-axis accelerations and three-axis angular velocities in the same manner as in the first embodiment, and is attached to a golf club 3 or a part of a user 2, for example, as illustrated in Fig. 2(A), Fig. 2(B) and Fig. 2(C).

In the same manner as in the first embodiment, the user 2 performs a swing action for hitting a golf ball 4 according to procedures illustrated in Fig. 3. While the user 2 performs the action of hitting the golf ball 4 according to the procedures illustrated in Fig. 3, the sensor unit 10 measures three-axis accelerations and three-axis angular velocities in a predetermined cycle (for example, 1 ms), and sequentially transmits measured data to the motion analysis apparatus 20.

The motion analysis apparatus 20 has an imaging function. In a case where a first state regarding swing motion of the user 2 is detected by using data measured by the sensor unit 10, the motion analysis apparatus automatically starts to capture moving images of the swing motion of the user 2 or to continuously capture still images of the swing motions, and sequentially stores captured images in a storage section built thereinto. In a case where a second state regarding the swing motion of the user 2 is detected by using data measured by the sensor unit 10, the motion analysis apparatus 20 automatically finishes the imaging. In other words, in the present embodiment, the user 2 can obtain images regarding the swing motion without performing an operation for imaging on the motion analysis apparatus 20.

In the same manner as in the first embodiment, the motion analysis apparatus 20 detects a specific action in the swing motion in which the user 2 has hit the ball with the golf club 3, by using the data measured by the sensor unit 10. The motion analysis apparatus 20 generates analysis information in which the captured image data is correlated with the specific action in the swing motion so as to present the analysis information to the user 2 by using an image or a sound. The motion analysis apparatus 20 may be, for example, a portable apparatus such as a smart phone, or a personal computer (PC).

1-2-2. Configuration of motion analysis system

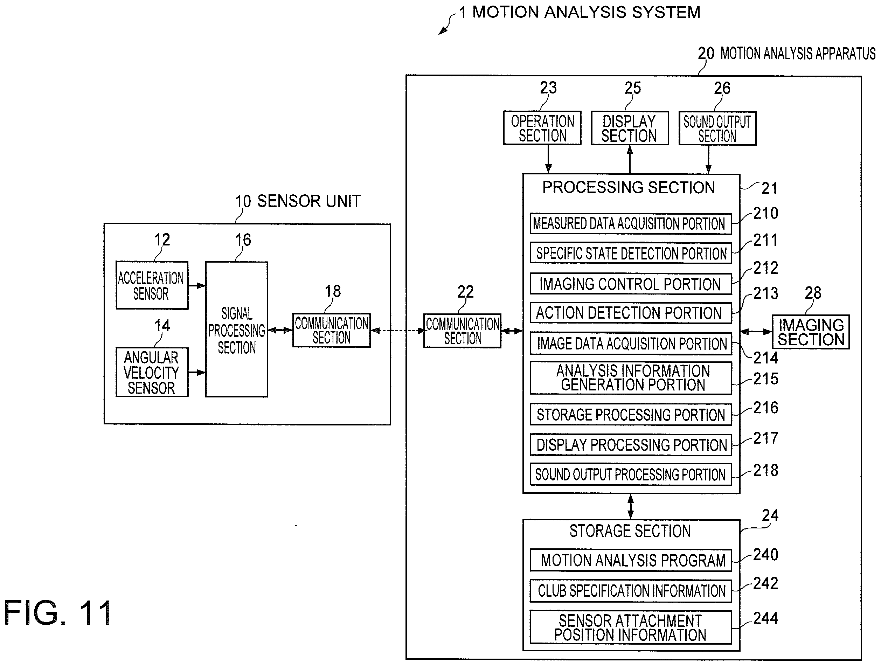

Fig. 11 is a diagram illustrating a configuration example of the motion analysis system 1 of the second embodiment. In Fig. 11, the same constituent elements as those in Fig. 4 are given the same reference numerals. As illustrated in Fig. 11, the motion analysis system 1 of the second embodiment is configured to include the sensor unit 10 and the motion analysis apparatus 20.

As illustrated in Fig. 11, a configuration and a function of the sensor unit 10 in the second embodiment are the same as those in the first embodiment. The motion analysis apparatus 20 in the second embodiment is configured to include a processing section 21, a communication section 22, an operation section 23, a storage section 24, a display section 25, a sound output section 26, and an imaging section 28 (an example of imaging means). Configurations and functions of the communication section 22, the operation section 23, the storage section 24, the display section 25, and the sound output section 26 are the same as those in the first embodiment.

The imaging section 28 performs a process of generating image data of moving images or still images corresponding to light emitted from a subject (user 2), and sending the generated image data to the processing section 21. For example, the imaging section 28 receives light emitted from the subject (user 2) with an imaging element (not illustrated) through a lens (not illustrated), converts the light into an electric signal, decomposes the electric signal into RGB components, and performs desired adjustment or correction and A/D conversion so as to generate image data.

If an instruction for capturing a still image is received from the processing section 21, the imaging section 28 generates image data of the still image. If an instruction for starting capturing of a moving image is received from the processing section 21, the imaging section 28 generates image data of the moving image at a set frame rate (for example, 60 frames/second). If an instruction for starting continuous capturing of still images is received from the processing section 21, the imaging section 28 continuously generates image data of the still images at a set time interval (for example, an interval of 0.1 seconds). If an instruction for finishing imaging is received from the processing section 21, generation of image data is finished.

The processing section 21 performs a process of transmitting a measurement control command to the sensor unit 10, or performs various computation processes on data which is received via the communication section 22 from the sensor unit 10. The processing section 21 performs a process of sending a control signal (imaging control command) for controlling imaging the imaging section 28, or performs various processes on data which is received from the imaging section 28. The processing section 21 performs other various control processes such as read/write processes of data for the storage section 24, a process of sending image data to the display section 25, and a process of sending sound data to the sound output section 26, according to operation data received from the operation section 23. Particularly, in the present embodiment, by executing the motion analysis program 240, the processing section 21 functions as a measured data acquisition portion 210, a specific state detection portion 211, an imaging control portion 212, an action detection portion 213, an image data acquisition portion 214, an analysis information generation portion 215, a storage processing portion 216, a display processing portion 217, and a sound output processing portion 218. Functions of the measured data acquisition portion 210, the specific state detection portion 211, the action detection portion 213, the analysis information generation portion 215, the storage processing portion 216, the display processing portion 217, and the sound output processing portion 218 are the same as those in the first embodiment.

In a case where the specific state detection portion 211 detects a specific state, the imaging control portion 212 performs a process of generating a control signal (imaging control command) for causing the imaging section 28 to control imaging, and sending the control signal to the imaging section 28. In the present embodiment, in a case where the specific state detection portion 211 detects the first state (a standing still state before the user 2 starts a swing), the imaging control portion 212 generates a first control signal (imaging starting command) for causing the imaging section 28 to start imaging, and sends the first control signal to the imaging section 28. In the present embodiment, in a case where the specific state detection portion 211 detects the second state (a standing still state after the user 2 finishes a swing), the imaging control portion 212 generates a second control signal (imaging finishing command) for causing the imaging section 28 to finish imaging, and sends the second control signal to the imaging section 28.

The image data acquisition portion 214 performs a process of acquiring image data captured by the imaging section 28. The image data acquired by the image data acquisition portion 214 are stored in the storage section 24 in correlation with measurement time points.

1-2-3. Imaging control

An example of imaging control performed by the processing section 21 in the second embodiment is the same as illustrated in Fig. 5, a diagram illustrating a correspondence relationship between image data and a flag is the same as Fig. 6, and thus illustration and description thereof will be omitted. However, in the second embodiment, the processing section 21 manages measurement time points and imaging time points, and thus measurement time points may also be used as the imaging time points. Since there is little communication delay between the processing section 21 and the imaging section 28, the measurement time point $t_2$ at which the processing section 21 detects a standing still state before starting a swing may be used as a time point at which the imaging section 28 starts imaging. Similarly, the measurement time point $t_9$ at which the processing section 21 detects a standing still state after starting the swing may be used as a time point at which the imaging section 28 finishes the imaging.

1-2-4. Process in motion analysis apparatus

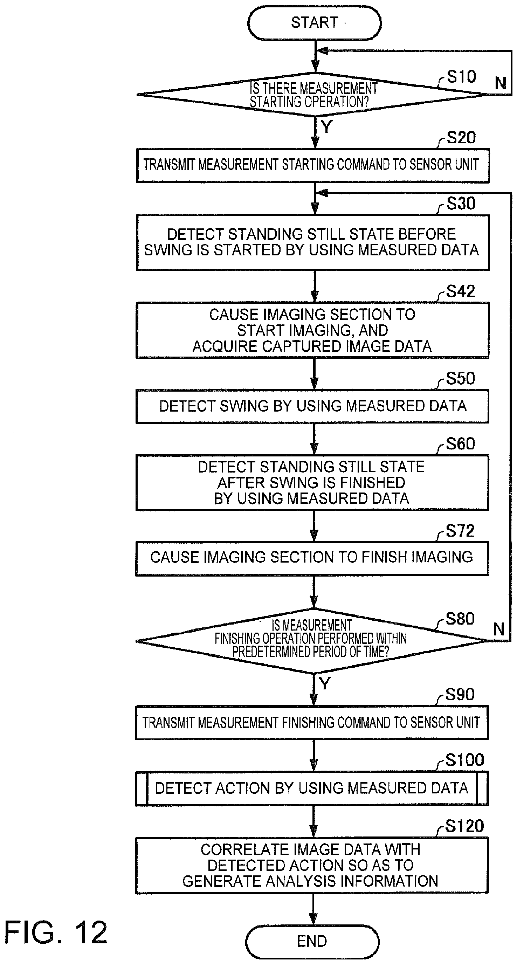

Fig. 12 is a flowchart illustrating examples of procedures of a motion analysis process performed by the processing section 21 in the second embodiment. In Fig. 12, steps in which the same processes are performed are given the same numbers as in Fig. 7. The processing section 21 performs the motion analysis process, for example, according to the procedures shown in the flowchart of Fig. 12 by executing the motion analysis program 240 stored in the storage section 24. Hereinafter, the flowchart of Fig. 12 will be described.

First, the processing section 21 determines whether or not a measurement starting operation has been performed on the basis of operation data (step S10), and waits for the measurement starting operation to be performed (N in step S10). In a case where the measurement starting operation has been performed (Y in step S10), the processing section 21 performs processes in steps S10 to S30 in the same manner as in Fig. 7, then sends an imaging starting command to the imaging section 28 so as to cause the imaging section 28 to start imaging, and acquires captured image data (step S42).

Next, the processing section 21 performs processes in steps S50 and S60 in the same manner as in Fig. 7, and then sends an imaging finishing command to the imaging section 28 so as to cause the imaging section 28 to finish the imaging (step S72).

Next, the processing section 21 determines whether or not a measurement finishing operation has been performed within a predetermined period of time on the basis of operation data (step S80), and performs the processes in step S30 and the subsequent steps again in a case where the measurement finishing operation has not been performed within the predetermined period of time (N in step S80).

In a case where the measurement finishing operation has been performed within the predetermined period of time (Y in step S80), the processing section 21 performs processes in steps S90 and S100 in the same manner as in Fig. 7, then generates analysis information in which the image data acquired in step S42 and each action detected in step S100 (step S120), and finishes the process.

In the flowchart of Fig. 12, order of the respective steps may be changed as appropriate within an allowable range.

1-2-5. Effects

According to the motion analysis system 1 of the second embodiment, it is possible to achieve the same effects as those in the first embodiment. In the motion analysis system 1 of the second embodiment, since communication delay between the processing section 21 and the imaging section 28 is almost neglected, for example, a measurement time point at which the processing section 21 detects a standing still state before starting a swing may be used as a time point at which the imaging section 28 starts imaging, and a measurement time point at which the processing section 21 detects a standing still state after starting the swing may be used as a time point at which the imaging section 28 finishes the imaging. Therefore, the motion analysis apparatus 20 can easily and accurately correlates captured image data with a detected action, and can thus provide highly accurate analysis information.

2. Modification Examples

The present invention is not limited to the present embodiment, and may be variously modified within the scope of the spirit of the present invention.

For example, in the above-described respective embodiments, the imaging control portion 212 causes imaging to be immediately started in a case where the specific state detection portion 211 detects the first state (for example, a standing still state before the user 2 starts a swing), but an imaging starting time point may be delayed by taking into consideration time from address to a top or impact in a case where a swing after the top swing, or the moment of the impact can be imaged. For example, when the user 2 performs a swing, a difference between a measurement time point at which the specific state detection portion 211 detects a standing still state before starting the swing and a measurement time point at which the action detection portion detects a top or impact may be computed, and time from address to the top or the impact may be predicted, for example, by obtaining an average value of differences in a plurality of latest swings performed by the user 2. In a case where the specific state detection portion 211 detects a standing still state (address) before a swing is started, the imaging control portion 212 may start imaging slightly before the top or the impact by taking into consideration the predicted time up to the top or the impact. In the above-described way, it is possible to considerably reduce an amount of image data and also to obtain images of a swing after the top, or images of the moment of the impact.

In the above-described respective embodiments, a standing still state before the user 2 starts a swing has been described as an example of the first state detected by the specific state detection portion 211, but the specific state detection portion 211 may detect swing starting or a top as the first state as long as the moment of impact can be imaged.

In the above-described respective embodiments, a standing still state after the user 2 finishes a swing has been described as an example of the second state detected by the specific state detection portion 211, but the specific state detection portion 211 may detect impact as the second state as long as the moment of the impact can be imaged.

In the above-described respective embodiments, in a case where the specific state detection portion 211 detects the first state (for example, a standing still state before the user 2 starts a swing), the imaging control portion 212 starts imaging, but may increase an imaging resolution. That is to say, in a case where the specific state detection portion 211 detects the first state, the imaging control portion 212 may generate a first control signal (a high-resolution setting command) for increasing an imaging resolution, and may transmits the signal to the imaging apparatus 30 or the imaging section 28. In the above-described way, for example, an amount of image data can be reduced by performing imaging at a low resolution before address, and a clear image can be obtained by performing imaging at a high resolution during a swing.

In the above-described respective embodiments, in a case where the specific state detection portion 211 detects the second state (for example, a standing still state after the user 2 finishes a swing), the imaging control portion 212 finishes imaging, but may reduce an imaging resolution. That is to say, in a case where the specific state detection portion 211 detects the second state, the imaging control portion 212 may generate a second control signal (a low-resolution setting command) for reducing an imaging resolution, and may transmits the signal to the imaging apparatus 30 or the imaging section 28. In the above-described way, for example, an amount of image data can be reduced by performing imaging at a low resolution after a swing is finished, and a clear image can be obtained by performing imaging at a high resolution during a swing.

In the above-described first embodiment, the motion analysis apparatus 20 detects a specific state by using measured data received from the sensor unit 10, and transmits the imaging control command to the imaging apparatus 30, but there may be a modification in which the sensor unit 10 has the functions of the specific state detection portion 211 and the imaging control portion 212, and transmits the imaging control command to the imaging apparatus 30 in a case of detecting the specific state. In the above-described way, since communication delay can be shortened, the moment of impact can be imaged even if imaging is started when a state (for example, a top in a swing) slightly before the impact is detected, and thus it is possible to reduce an amount of captured image data.

In the above-described respective embodiments, a timing (impact) at which the user 2 has hit the ball is detected by using the square root of the square sum as shown in Equation (2) as a combined value of three-axis angular velocities measured by the sensor unit 10, but, as a combined value of three-axis angular velocities, for example, a square sum of three-axis angular velocities, a sum or an average of three-axis angular velocities, or the product of three-axis angular velocities may be used. Instead of a combined value of three-axis angular velocities, a combined value of three-axis accelerations such as a square sum or a square root of three-axis accelerations, a sum or an average value of three-axis accelerations, or the product of three-axis accelerations may be used.

In the above-described respective embodiments, the acceleration sensor 12 and the angular velocity sensor 14 are built into and are thus integrally formed as the sensor unit 10, but the acceleration sensor 12 and the angular velocity sensor 14 may not be integrally formed. Alternatively, the acceleration sensor 12 and the angular velocity sensor 14 may not be built into the sensor unit 10, and may be directly mounted on the golf club 3 or the user 2. In the above-described embodiments, the sensor unit 10 and the motion analysis apparatus 20 are separately provided, but may be integrally formed so as to be attached to the golf club 3 or the user 2.

In the above-described embodiments, golf has been exemplified as an example of a sport done by the user 2, but the present invention is applicable to various sports such as tennis or baseball. For example, the sensor unit 10 may be attached to a baseball bat, the motion analysis apparatus 20 may detect the moment of ball hitting on the basis of a change in acceleration, and the imaging apparatus 30 (or the motion analysis apparatus 20 having an imaging function) may perform imaging right after the ball hitting. The present invention is also applicable to various sports not requiring a swing action, such as skiing or snowboarding. For example, the sensor unit 10 and the imaging apparatus 30 (or the motion analysis apparatus 20 having an imaging function) may be attached to a ski jumper, the motion analysis apparatus 20 may detect the highest point on the basis of a change in acceleration or the like, and the imaging apparatus 30 (or the motion analysis apparatus 20) may perform imaging at the highest point. Alternatively, the sensor unit 10 may be attached to a snowboard, the motion analysis apparatus 20 may detect impact on the basis of a change in acceleration or the like, and the imaging apparatus 30 (or the motion analysis apparatus 20 having an imaging function) may perform imaging at a timing at which the snowboard comes close to a snow surface.

The above-described embodiments and modification examples are only examples, and the present invention is not limited thereto. For example, the respective embodiments and the respective modification examples may be combined with each other as appropriate.

For example, the present invention includes substantially the same configuration (for example, a configuration in which functions, methods, and results are the same, or a configuration in which objects and effects are the same) as the configuration described in the embodiments. The present invention includes a configuration in which an inessential part of the configuration described in the embodiments is replaced with another part. The present invention includes a configuration which achieves the same operation and effect or a configuration capable of achieving the same object as in the configuration described in the embodiments. The invention includes a configuration in which a well-known technique is added to the configuration described in the embodiment.

Reference Signs List

1 MOTION ANALYSIS SYSTEM, 2 USER, 3 GOLF CLUB, 4 GOLF BALL, 10 SENSOR UNIT, 12 ACCELERATION SENSOR, 14 ANGULAR VELOCITY SENSOR, 16 SIGNAL PROCESSING SECTION, 18 COMMUNICATION SECTION, 20 MOTION ANALYSIS APPARATUS, 21 PROCESSING SECTION, 22 COMMUNICATION SECTION, 23 OPERATION SECTION, 24 STORAGE SECTION, 25 DISPLAY SECTION, 26 SOUND OUTPUT SECTION, 27 COMMUNICATION SECTION, 28 IMAGING SECTION, 30 IMAGING APPARATUS, 31 PROCESSING SECTION, 32 COMMUNICATION SECTION, 33 OPERATION SECTION, 34 STORAGE SECTION, 35 DISPLAY SECTION, 36 IMAGING SECTION, 210 MEASURED DATA ACQUISITION PORTION, 211 SPECIFIC STATE DETECTION PORTION, 212 IMAGING CONTROL PORTION, 213 ACTION DETECTION PORTION, 214 IMAGE DATA ACQUISITION PORTION, 215 ANALYSIS INFORMATION GENERATION PORTION, 216 STORAGE PROCESSING PORTION, 217 DISPLAY PROCESSING PORTION, 218 SOUND OUTPUT PROCESSING PORTION, 240 MOTION ANALYSIS PROGRAM, 242 CLUB SPECIFICATION INFORMATION, 244 SENSOR ATTACHMENT POSITION INFORMATION

**Claims**

1.  A motion analysis method comprising:

     an action detection step of detecting an action in motion of a user by using an output signal from an inertial sensor;
     an image data acquisition step of acquiring image data of the motion of the user, captured by imaging means; and
     an analysis information generation step of correlating the image data with the detected action.

2.  The motion analysis method according to claim 1, wherein synchronization between the output signal from the inertial sensor and the image data captured by the imaging means occurs.

3.  The motion analysis method according to claim 2, wherein the synchronization occurs on the basis of a timing of a first state in the motion, detected on the basis of the output signal from the inertial sensor.

4.  The motion analysis method according to any one of claims 1 to 3,
     wherein, in the analysis information generation step, a flag is added to image data corresponding to the detected action among the image data items.

5.  The motion analysis method according to claim 4, wherein, in the analysis information generation step, types of the flag are different from each other depending on types of the detected action.

6.  The motion analysis method according to any one of claims 1 to 5,
     wherein the motion is swing motion using an exercise appliance.

7.  The motion analysis method according to claim 6, wherein, in the action detection step, an action when the user starts the swing motion is detected.

8.  The motion analysis method according to claim 6 or 7,
     wherein, in the action detection step, an action when the user switches a direction of the swing motion is detected.

9.  The motion analysis method according to any one of claims 6 to 8,
     wherein, in the action detection step, an action when a speed of the swing motion is the maximum is detected.

10. The motion analysis method according to any one of claims 6 to 9,
     wherein, in the action detection step, an action when the user hits a ball is detected.

11. The motion analysis method according to any one of claims 6 to 10, wherein, in the action detection step, an action when the user finishes the swing motion is detected.

12. The motion analysis method according to any one of claims 6 to 11, wherein the exercise appliance is a golf club.

13. The motion analysis method according to any one of claims 1 to 12, wherein imaging performed by the imaging means is continuous imaging of still images.

14. A motion analysis apparatus comprising:

an action detection portion that detects an action in motion of a user by using an output signal from an inertial sensor; an image data acquisition portion that acquires image data of the motion of the user, captured by imaging means; and an analysis information generation portion that correlates the image data with the detected action.

15. The motion analysis apparatus according to claim 14, wherein the analysis information generation portion adds a flag to image data corresponding to the detected action among the image data items.

16. The motion analysis apparatus according to claim 15, wherein the analysis information generation portion generates different types of the flag depending on types of the detected action.

17. The motion analysis apparatus according to claim 14, wherein the motion is swing motion using an exercise appliance.

18. The motion analysis apparatus according to claim 17, wherein the action detection portion detects an action when the user starts the swing motion.

19. A motion analysis system comprising:

the motion analysis apparatus according to claim 14; and the inertial sensor.

20. The motion analysis system according to claim 19, further comprising the imaging means.

1 MOTION ANALYSIS SYSTEM

20 MOTION ANALYSIS APPARATUS

10 SENSOR UNIT

30 IMAGING APPARATUS

FIG. 1

# FIG. 2A

# FIG. 2B

# FIG. 2C

EP 3 181 203 A1

START

S1

TAKE ADDRESS ATTITUDE SO THAT LONG AXIS
OF SHAFT OF GOLF CLUB IS
PERPENDICULAR TO TARGET LINE, AND STAND
STILL FOR PREDETERMINED TIME OR MORE

S2

PERFORM SWING ACTION SO AS TO HIT BALL

END

FIG. 3

FIG. 4

1 MOTION ANALYSIS SYSTEM

20 MOTION ANALYSIS APPARATUS

23 OPERATION SECTION

25 DISPLAY SECTION

26 SOUND OUTPUT SECTION

30 IMAGING APPARATUS

10 SENSOR UNIT

PROCESSING SECTION — 21

MEASURED DATA ACQUISITION PORTION — 210

SPECIFIC STATE DETECTION PORTION — 211

IMAGING CONTROL PORTION — 212

ACTION DETECTION PORTION — 213

IMAGE DATA ACQUISITION PORTION — 214

ANALYSIS INFORMATION GENERATION PORTION — 215

STORAGE PROCESSING PORTION — 216

DISPLAY PROCESSING PORTION — 217

SOUND OUTPUT PROCESSING PORTION — 218

12 ACCELERATION SENSOR

16 SIGNAL PROCESSING SECTION

18 COMMUNICATION SECTION

22 COMMUNICATION SECTION

27 COMMUNICATION SECTION

32 COMMUNICATION SECTION

36 IMAGING SECTION

31 PROCESSING SECTION

33 OPERATION SECTION

35 DISPLAY SECTION

34 STORAGE SECTION

14 ANGULAR VELOCITY SENSOR

STORAGE SECTION — 24

MOTION ANALYSIS PROGRAM — 240

CLUB SPECIFICATION INFORMATION — 242

SENSOR ATTACHMENT POSITION INFORMATION — 244

EP 3 181 203 A1

FIG. 5

IMAGING TIME POINT: $T_0$ | IMAGE DATA 0

IMAGING TIME POINT: $T_{104}$ | IMAGE DATA 104

SWING STARTING → IMAGING TIME POINT: $T_{105}$ | IMAGE DATA 105 — FLAG 1

IMAGING TIME POINT: $T_{106}$ | IMAGE DATA 106

IMAGING TIME POINT: $T_{189}$ | IMAGE DATA 189

TOP → IMAGING TIME POINT: $T_{190}$ | IMAGE DATA 190 — FLAG 2

IMAGING TIME POINT: $T_{191}$ | IMAGE DATA 191

IMAGING TIME POINT: $T_{239}$ | IMAGE DATA 239

NATURAL UNCOCK → IMAGING TIME POINT: $T_{240}$ | IMAGE DATA 240 — FLAG 3

IMAGING TIME POINT: $T_{241}$ | IMAGE DATA 241

IMAGING TIME POINT: $T_{249}$ | IMAGE DATA 249

IMPACT → IMAGING TIME POINT: $T_{250}$ | IMAGE DATA 250 — FLAG 4

IMAGING TIME POINT: $T_{251}$ | IMAGE DATA 251

IMAGING TIME POINT: $T_{304}$ | IMAGE DATA 304

SWING FINISHING → IMAGING TIME POINT: $T_{305}$ | IMAGE DATA 305 — FLAG 5

FIG. 6    IMAGING TIME POINT: $T_{306}$ | IMAGE DATA 306

```
                    ┌─────────────┐
                    │    START    │
                    └─────────────┘
                           │
                           ▼
               ╱IS THERE MEASUREMENT╲─── S10  N
               ╲  STARTING OPERATION?╱
                           │ Y
                           ▼            S20
        ┌──────────────────────────────────────┐
        │ TRANSMIT MEASUREMENT STARTING COMMAND │
        │           TO SENSOR UNIT              │
        └──────────────────────────────────────┘
                           │                     S30
                           ▼
        ┌──────────────────────────────────────┐
        │ DETECT STANDING STILL STATE BEFORE    │
        │ SWING IS STARTED BY USING MEASURED DATA│
        └──────────────────────────────────────┘
                           │              S40
                           ▼
        ┌──────────────────────────────────────┐
        │ TRANSMIT IMAGING STARTING COMMAND     │
        │       TO IMAGING APPARATUS            │
        └──────────────────────────────────────┘
                           │              S50
                           ▼
        ┌──────────────────────────────────────┐
        │  DETECT SWING BY USING MEASURED DATA  │
        └──────────────────────────────────────┘
                           │              S60
                           ▼
        ┌──────────────────────────────────────┐
        │ DETECT STANDING STILL STATE AFTER     │
        │ SWING IS FINISHED BY USING MEASURED DATA│
        └──────────────────────────────────────┘
                           │              S70
                           ▼
        ┌──────────────────────────────────────┐
        │ TRANSMIT IMAGING FINISHING COMMAND    │
        │       TO IMAGING APPARATUS            │
        └──────────────────────────────────────┘
                           │
                           ▼               S80
               ╱IS MEASUREMENT FINISHING╲─── N
               ╲OPERATION PERFORMED WITHIN╱
                ╲PREDETERMINED PERIOD OF TIME?╱
                           │ Y
                           ▼              S90
        ┌──────────────────────────────────────┐
        │ TRANSMIT MEASUREMENT FINISHING COMMAND│
        │           TO SENSOR UNIT              │
        └──────────────────────────────────────┘
                           │              S100
                           ▼
        ┌──────────────────────────────────────┐
        │ DETECT EACH ACTION BY USING MEASURED  │
        │              DATA                     │
        └──────────────────────────────────────┘
                           │              S110
                           ▼
        ┌──────────────────────────────────────┐
        │ ACQUIRE IMAGE DATA FROM IMAGING       │
        │            APPARATUS                  │
        └──────────────────────────────────────┘
                           │              S120
                           ▼
        ┌──────────────────────────────────────┐
        │ CORRELATE IMAGE DATA WITH EACH        │
        │ DETECTED ACTION SO AS TO GENERATE     │
        │        ANALYSIS INFORMATION           │
        └──────────────────────────────────────┘
                           │
                           ▼
                    ┌─────────────┐
                    │     END     │
                    └─────────────┘
```

FIG. 7

START

S200
PERFORM BIAS CORRECTION ON MEASURED DATA

S210
COMPUTE COMBINED VALUE $n_0(t)$ OF ANGULAR VELOCITIES

S220
CONVERT COMBINED VALUE $n_0(t)$ INTO NORMALIZED COMBINED VALUE $n(t)$

S230
COMPUTE DERIVATIVE $dn(t)$ OF COMBINED VALUE $n(t)$

S240
OF TIME POINTS AT WHICH DERIVATIVE $dn(t)$ OF COMBINED VALUE IS MAXIMUM AND MINIMUM, SPECIFY EARLIER TIME POINT AS IMPACT MEASUREMENT TIME POINT $t_7$

S250
SPECIFY TIME POINT OF MINIMUM POINT AT WHICH $n(t)$ IS CLOSE TO 0 BEFORE MEASUREMENT TIME POINT $t_7$ AS TOP MEASUREMENT TIME POINT $t_5$

S260
SPECIFY TIME POINT OF MINIMUM POINT AT WHICH $n(t)$ IS CLOSE TO 0 AFTER MEASUREMENT TIME POINT $t_7$ AS SWING FINISHING MEASUREMENT TIME POINT $t_8$

S270
SPECIFY INTERVAL IN WHICH $n(t)$ IS EQUAL TO OR SMALLER THAN PREDETERMINED THRESHOLD VALUE BEFORE AND AFTER MEASUREMENT TIME POINT $t_5$ AS TOP INTERVAL

S280
SPECIFY LAST TIME POINT AT WHICH $n(t)$ IS EQUAL TO OR SMALLER THAN PREDETERMINED THRESHOLD VALUE BEFORE STARTING TIME POINT OF TOP INTERVAL AS SWING STARTING MEASUREMENT TIME POINT $t_4$

S290
COMPUTE GRIP SPEED $v(t)$

S300
SPECIFY TIME POINT AT WHICH GRIP SPEED $v(t)$ IS MAXIMUM AS MEASUREMENT TIME POINT $t_6$ OF NATURAL UNCOCK

END

FIG. 8

FIG. 9A

ANGULAR
VELOCITY
[dps]

FIG. 9B

COMBINED VALUE OF
ANGULAR VELOCITIES
(NORMALIZED TO 0 TO 100)

FIG. 9C

DERIVATIVE OF
COMBINED VALUE OF
ANGULAR VELOCITIES

2

1 MOTION ANALYSIS SYSTEM

10 SENSOR UNIT

3

4

20 MOTION ANALYSIS APPARATUS

FIG. 10

FIG. 11

1 MOTION ANALYSIS SYSTEM

20 MOTION ANALYSIS APPARATUS

23 OPERATION SECTION

25 DISPLAY SECTION

26 SOUND OUTPUT SECTION

10 SENSOR UNIT

12 ACCELERATION SENSOR

14 ANGULAR VELOCITY SENSOR

16 SIGNAL PROCESSING SECTION

18 COMMUNICATION SECTION

22 COMMUNICATION SECTION

PROCESSING SECTION — 21

MEASURED DATA ACQUISITION PORTION — 210

SPECIFIC STATE DETECTION PORTION — 211

IMAGING CONTROL PORTION — 212

ACTION DETECTION PORTION — 213

IMAGE DATA ACQUISITION PORTION — 214

ANALYSIS INFORMATION GENERATION PORTION — 215

STORAGE PROCESSING PORTION — 216

DISPLAY PROCESSING PORTION — 217

SOUND OUTPUT PROCESSING PORTION — 218

28 IMAGING SECTION

STORAGE SECTION — 24

MOTION ANALYSIS PROGRAM — 240

CLUB SPECIFICATION INFORMATION — 242

SENSOR ATTACHMENT POSITION INFORMATION — 244

EP 3 181 203 A1

START

S10 — IS THERE MEASUREMENT STARTING OPERATION? — N

Y

S20 — TRANSMIT MEASUREMENT STARTING COMMAND TO SENSOR UNIT

S30 — DETECT STANDING STILL STATE BEFORE SWING IS STARTED BY USING MEASURED DATA

S42 — CAUSE IMAGING SECTION TO START IMAGING, AND ACQUIRE CAPTURED IMAGE DATA

S50 — DETECT SWING BY USING MEASURED DATA

S60 — DETECT STANDING STILL STATE AFTER SWING IS FINISHED BY USING MEASURED DATA

S72 — CAUSE IMAGING SECTION TO FINISH IMAGING

S80 — IS MEASUREMENT FINISHING OPERATION PERFORMED WITHIN PREDETERMINED PERIOD OF TIME? — N

Y

S90 — TRANSMIT MEASUREMENT FINISHING COMMAND TO SENSOR UNIT

S100 — DETECT ACTION BY USING MEASURED DATA

S120 — CORRELATE IMAGE DATA WITH DETECTED ACTION SO AS TO GENERATE ANALYSIS INFORMATION

END

FIG. 12

## INTERNATIONAL SEARCH REPORT

International application No.

PCT/JP2015/003953

A. CLASSIFICATION OF SUBJECT MATTER

*A63B69/36(2006.01)i*

According to International Patent Classification (IPC) or to both national classification and IPC

B. FIELDS SEARCHED

Minimum documentation searched (classification system followed by classification symbols)

A63B69/36, A61B5/22, A61B5/103-5/117

Documentation searched other than minimum documentation to the extent that such documents are included in the fields searched

| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2005-110850 A (Hitachi Metals, Ltd., Keio University), 28 April 2005 (28.04.2005), paragraphs [0026] to [0067]; fig. 1 to 15 (Family: none) | 1-20 |
| X Y | JP 2009-106323 A (Yamaha Corp.), 21 May 2009 (21.05.2009), paragraphs [0015] to [0043]; fig. 1 to 4 (Family: none) | 1-2,14-20 3-13 |
| X Y | JP 2013-202066 A (Seiko Epson Corp.), 07 October 2013 (07.10.2013), paragraphs [0025] to [0073]; fig. 1 to 14 (Family: none) | 1-7,14-20 8-13 |

☒ Further documents are listed in the continuation of Box C.      ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 27 October 2015 (27.10.15) | 10 November 2015 (10.11.15) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (July 2009)

**INTERNATIONAL SEARCH REPORT**

International application No.

PCT/JP2015/003953

C (Continuation).  DOCUMENTS CONSIDERED TO BE RELEVANT

| Category* | Citation of document, with indication, where appropriate, of the relevant passages | Relevant to claim No. |
|---|---|---|
| Y | JP 6-39070 A  (Mitsui Bussan Digital Kabushiki Kaisha),<br>15 February 1994 (15.02.1994),<br>paragraphs [0022] to [0029]; fig. 2 to 4<br>(Family: none) | 7-13 |

Form PCT/ISA/210 (continuation of second sheet) (July 2009)

**EP 3 181 203 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008073210 A